# EUROPEAN PATENT SPECIFICATION

(11) **EP 3 934 577 B1**
(45) Date of publication and mention of the grant of the patent: **30.07.2025**
(21) Application number: 20766968.0
(22) Date of filing: 21.02.2020
(51) Int. Cl.: A61F 2/02, A61N 1/375, A61L 31/14, A61L 31/04, A61L 27/40, A61L 27/50, A61F 2/00, A61L 27/34, A61L 27/46, A61L 27/48, A61L 27/56, A61L 27/58, A61N 1/05

(54) **A CONFORMABLE COVER FOR A MEDICAL DEVICE**
EINE ANPASSUNGSFÄHIGE ABDECKUNG FÜR EIN MEDIZINISCHES GERÄT.
UNE COUVERTURE ADAPTABLE POUR UN DISPOSITIF MÉDICAL.

(30) Priority: 04.03.2019 AU 2019900699
(43) Date of publication of application: 12.01.2022
(73) Proprietor: GLOBAL SURGICAL INNOVATIONS PTY LTD, Burwood, NSW 2134 (AU)
(72) Inventor: BOUGHTON, Elizabeth Anne, Burwood, New South Wales 2134 (AU); BOUGHTON, Philip, Burwood, New South Wales 2134 (AU)
(74) Representative: Loo, Chi Ching
(86) International application number: PCT/AU2020/050157
(87) International publication number: WO 2020/176923

(56) References cited:
- WO-A1-2018/148722
- WO-A1-2018/148722
- US-A1- 2010 168 808
- US-A1- 2010 168 808
- US-A1- 2011 238 094
- US-A1- 2011 238 094
- US-A1- 2012 059 467
- US-B2- 9 848 955
- US-B2- 9 848 955

## Description

### Field of the Invention

The present invention relates to a conformable cover for a medical device.

### Background of the Invention

All materials elicit a biological response when implanted in living tissues. When a nontoxic biologically inactive (or inert) material is implanted, the living tissue will respond by forming a fibrous capsule of variable thickness around the implant. The thickness of the capsule formed depends on the type of material implanted and the extent of relative motion between the implant and the tissue. The formation of a relatively thick capsule generally leads to implant loosening, and subsequent clinical failure. This is especially a problem in soft tissue implants, because mechanical fixation is not able to be achieved to the same extent as in hard tissue. Failure originating from the material-tissue interface is one of the most common reasons for the need to revise medical implants.

'Silastic' materials refer to a group of polymeric silicone substances that possess rubber-like characteristics and are biologically inert, and for these characteristics, are commonly used in implantable medical devices. Silastic materials have been used as the implantable soft tissue interface portion of various medical devices. As with other biologically inert materials, silastic materials, when implanted, causes the patient's body to react by encasing the implanted material in fibrous tissue, and subsequently scar tissue.

The present invention seeks to overcome or substantially ameliorate at least some of the deficiencies of the prior art in implantable medical devices, or to at least provide an alternative. Covers for implantable medical devices are known from the documents US-A-2012/059467 and US-A-9848955.

### Summary of the Invention

The invention is set out in the appended set of claims. According to a first aspect not in accordance with the invention, an attachment means for attaching a medical device to tissue is provided, the attachment means comprising:
- a biocompatible body comprising a body polymer component; and
- a biocompatible scaffold comprising a scaffold polymer component, the biocompatible scaffold extending from at least one surface of the biocompatible body.

Advantageously, the attachment means provides a fixation mechanism for a medical device to be at least partially implanted within the soft tissue of a patient. The attachment means, once implanted into the soft tissue of the patient, encourages the soft tissue cells to infiltrate into the biocompatible scaffold and for vascularisation to occur within the biocompatible scaffold. Once the cells and vasculature have sufficiently infiltrated into the biocompatible scaffold, a cellular-scaffold bio-adhesion and mechanical interlock is achieved, substantially fixing the attachment means and the medical device to the patient.

The body polymer component may be biostable.

The body polymer component may be bioresorbable.

The scaffold polymer component may be bioresorbable.

Advantageously, as the biocompatible scaffold gradually resorbs within the in vivo environment, the infiltrated soft tissue grows to replace the biocompatible scaffold. When the biocompatible scaffold has completely resorbed, the surrounding host tissue provides a relatively tight mechanical fixation of the biocompatible body within the patient. In this way, the biocompatible scaffold can be described as mediating a stable, mechanical implant- tissue bridge.

Advantageously, the more stably and firmly fixed an implant is within the host tissue, the lesser will be the inflammatory response of the host tissue, and thus the lower the likelihood of the host tissue developing a fibrous capsule or scar tissue. As the development of a thick fibrous capsule within the host tissue can potentially lead to clinical failure, use of the attachment means for attaching at least partially implantable medical devices to soft tissue substantially provides for the long-term clinical success of the medical device.

The biocompatible scaffold may at least partially cover two or more surfaces of the biocompatible body.

The biocompatible scaffold may at least partially cover two or more opposing surfaces of the biocompatible body.

The biocompatible scaffold may be located on the surfaces of the biocompatible body that comes into contact with the host tissue of the patient when implanted.

At least a portion of the biocompatible scaffold may be impregnated within at least a portion of the biocompatible body.

Advantageously, there exists a graded region comprising both the biocompatible scaffold and the biocompatible body. The host tissue is capable of replacing the biocompatible scaffold by infiltrating into the small spaces within the biocompatible body previously occupied by the biocompatible scaffold. When the biocompatible scaffold is fully resorbed, the infiltration and vascularisation of tissue into the biocompatible body provides a strong, stable, long-term, mechanical implant-tissue interlock.

The biocompatible scaffold may extend through the entire thickness of at least a portion of the biocompatible body.

Advantageously, tissue ingrowth and vascularisation can occur through the entire thickness of the biocompatible body, substantially improving the long-term strength of the mechanical implant-tissue fixation.

The biocompatible scaffold may further comprise a bioactive material.

For example, the bioactive material may comprise hydroxyapatite or carbonated apatite or calcium carbonate or calcium carbonate.

The bioactive material may comprise bioactive glass including one or more of hydroxyapatite or carbonated apatite or calcium carbonate or calcium carbonate.

The body polymer component may comprise a plurality of macro-diameter canals extending from at least one outer surface to an opposing outer surface.

Advantageously, the host tissue is able to infiltrate and vascularise within the canals and through the biocompatible polymer body, providing an additional mechanism to improve the strength of the mechanical implant-tissue fixation.

Each of the macro-diameter canals may be substantially rounded in cross- section along it entire length.

Advantageously, canals that are rounded do not create undue stress concentration that weakens the biocompatible body.

The macro-diameter canals may be within a range of 1 mm to 15 mm in diameter.

The macro-diameter canals may be within a range of3 mm to 10 mm in diameter.

Advantageously, the canals are sized to encourage tissue infiltration and vascularisation.

The biocompatible body may further comprise a body bioactive glass component.

Certain compositions of bioactive glass promote soft tissue growth and adhesion *in vivo.* The presence of bioactive glass within the canals actively encourages soft tissue infiltration and adhesion within the canals in use, and also prevents the formation of fibrous and scar tissue.

The body bioactive glass component may be a bioactive glass lining within the macro-diameter canals.

The body bioactive glass component may be a bioactive glass filler within the macro-diameter canals.

The body bioactive glass component may be a bioactive glass coating on at least one surface of the body polymer component.

The body bioactive glass component may be in the form of microparticles.

The body bioactive glass component may include hydroxyapatite.

The body bioactive glass component may include carbonated apatite.

The body bioactive glass component may include calcium carbonate.

Advantageously, microparticles, having relatively small diameters, do not create an excessively bumpy topography (in comparison with the topography larger particles would produce). Thus, when the body bioactive glass component is in contact with the host tissue, minimal irritation and abrasion is caused by the rubbing against the tissue. Microparticles are also less likely to cause stress concentrations and form cracks in the surface of the biocompatible body.

The body bioactive glass component may be in the form of microspheres or microbeads.

Spherical glass particles scatter light, which is an especially advantageous property in embodiments in which bioactive glass is present within the macro-diameter canals. As the canals extend from at least one surface to an opposing surface of the biocompatible body, the spherical bioactive glass particles help light applied at one surface travel through the biocompatible body to its opposing surface.

The microspheres or microbeads may be hollow.

The microparticles may be equal to or less than 30 µm in diameter.

The microparticles may be equal to or less than 10 µm in diameter.

The microparticles may be equal to or less than 1 µm in diameter.

The body polymer component may be made of a biocompatible elastomer.

The biocompatible elastomer may be a silastic material.

The biocompatible elastomer may be a polyurethane material.

The body polymer component may be made of a polycarbonate material.

The body polymer component may have a high transparency.

In one embodiment, the application of light to the biocompatible scaffold and/or the biocompatible body may form a part of the method of using the attachment means to attach a medical device to tissue. In this embodiment, the transparency of the body polymer component allows light applied to the biocompatible scaffold covering one side of the biocompatible body to travel through the biocompatible body to reach the biocompatible scaffold covering another side of the biocompatible body. Thus, even underlying portions of the biocompatible scaffold and biocompatible body can be exposed to the applied light.

The biocompatible scaffold may comprise a network of interconnective micropores.

The network of interconnective micropores within the biocompatible scaffold provides the necessary pathways through which cellular integration, vascularisation, oxygenation and toxin drainage can occur.

The network of interconnective micropores may have a non-repeating, randomised structure.

Advantageously, a non-repeating, randomised structure provides pathways through which blood vessels can more naturally navigate, in comparison with the more tortuous paths provided by a repeating structure.

The network of interconnective micropores may comprise a plurality of relatively small micropores located within a plurality of relatively large micropores.

Advantageously, the small micropores provide adhesion sites for cells and the larger micropores allow vascularisation, toxin drainage and tissue growth.

A plurality of the interconnective micropores may be within the range of 50 µm to 600 µm in diameter.

A plurality of the interconnective micropores may be within the range of 60 µm to 500 µm in diameter.

A plurality of the interconnective micropores may be within the range of 1 µm to 20 µm in diameter.

A plurality of the interconnective micropores may be within the range of 5 µm to 15 µm in diameter.

The biocompatible scaffold may have a porosity of or greater than 80vol%.

The biocompatible scaffold may have a porosity of or greater than 90vol%.

The biocompatible scaffold may have a porosity of or greater than 95vol%.

Advantageously, a scaffold structure having a high porosity allows cells and tissue to infiltrate and navigate with very little obstruction while still providing a framework on which the tissues can anchor and be supported.

A highly porous biocompatible scaffold is mechanically advantageous, as it is soft, compressible and extensible, avoiding the production of stress concentration which may cause damage to the surrounding tissue in use. Being compressible and extensible also allows the biocompatible scaffold to conform intimately to the tissue interface to ensure reliable contact and adhesion. These factors reduce irritation, the likelihood of infection and resultant inflammatory response at the implant-tissue interface, which in turn reduces the likelihood that a thick fibrous, fluid filled capsule will be formed to isolate the implant from the surrounding tissue.

The biocompatible scaffold may be a non-woven pile.

Advantageously, this is a type of structure that will provide a highly porous scaffold having a network of interconnective, randomised, non-repeating micropores.

The biocompatible scaffold may be a felt structure.

Advantageously, this is a type of structure that will provide a highly porous scaffold having a network of interconnective, randomised, non-repeating micropores.

The biocompatible scaffold may be a 3D fabric.

Advantageously, this is a type of structure that will provide a highly porous scaffold having a network of interconnective micropores.

The biocompatible scaffold may be a 3D mesh.

Advantageously, this is a type of structure that will provide a highly porous scaffold having a network of interconnective micropores.

The scaffold polymer component may be made of an aliphatic polyester thermoplastic.

The aliphatic polyester thermoplastic may have a medium to high molecular weight.

The scaffold polymer component may be made of a polypropylene.

The scaffold polymer component may comprise a high molecular weight chitosan-based polymer.

The scaffold polymer component may comprise a low molecular weight chitosan-based polymer.

The scaffold polymer component may comprise carboxymethylcellulose.

The scaffold polymer component may comprise suitable variants of carboxymethylcellulose.

In other embodiments, the scaffold polymer component may comprise biodegradeable polyurethane or polylactic acid (PLA) or poly-L/D-lactide (PLDLA) or polyvinyl alcohol (PVA).

A bonding property of the biocompatible scaffold may be capable of being activated in use.

Advantageously, the light-activated bonding provides a mechanism by which the biocompatible scaffold can be initially adhered to the surrounding tissue upon implantation. This initial adherence substantially immobilises the attachment means within the host tissue at an early time point, and facilitates the slower, longer term fixation of tissue infiltration into the biocompatible scaffold and the surface of the biocompatible polymer body.

The biocompatible scaffold may further comprise a scaffold crosslinking component capable of crosslinking to tissue in use when activated.

Advantageously, scaffold-tissue crosslinking is a relatively strong mechanism for initially bonding the biocompatible scaffold (and therefore the attachment means) to the host tissue.

The crosslinking component may be activated by light energy.

The crosslinking component may be activated by heat.

The crosslinking component may be activated by the body heat of the patient or a subject

Advantageously, the crosslinking is spontaneously activated when the attachment means is implanted into the patient.

The scaffold crosslinking component may crosslink to tissue within 5 minutes of activation.

In another embodiment, the scaffold crosslinking component may crosslink to tissue within 3 minutes of activation.

In yet another embodiment, the scaffold crosslinking component may crosslink to tissue within 2 minutes of activation.

In another embodiment, the scaffold crosslinking component may crosslink to tissue within 1 minute of activation.

Advantageously, initial bonding between the biocompatible scaffold and adjacent host tissue can be achieved almost instantaneously following implantation.

The scaffold crosslinking component may be made of a photoactive agent.

The photoactive agent may be a chitosan film.

Advantageously, chitosan comprises an initiator that frees up chemical bonds upon application of light energy. The liberated chemical bonds rapidly crosslink to the collagen component of cells. Chitosan film is also capable of forming a strong mechanical anchor to the surfaces of the biocompatible scaffold.

The photoactive agent may be a synthetic polymer chitosan equivalent.

The scaffold crosslinking component may be made of a heat-active agent.

The heat-active agent may be a polyvinyl alcohol gel.

Advantageously, polyvinyl alcohol gel crosslinks to tissue when heated to body temperature.

The biocompatible scaffold may further comprises a scaffold bioactive glass component.

Certain compositions of bioactive glass promote soft tissue growth and adhesion *in vivo.* The presence of bioactive glass within the scaffold will actively encourage soft tissue infiltration into and adhesion to the biocompatible scaffold. The adhesion of the tissue to the scaffold *in vivo* prevents or substantially reduces the formation of fibrous and scar tissue.

The scaffold bioactive glass component may be impregnated within the scaffold polymer component.

The scaffold bioactive glass component may be coated on the scaffold polymer component.

The scaffold bioactive glass component may be in the form of microparticles.

Advantageously, microparticles, having relatively small diameters, do not create an excessively bumpy topography (in comparison with the topography larger particles would produce). Thus, when the scaffold bioactive glass component is in contact with the host tissue, minimal irritation and abrasion is caused by rubbing against the tissue. Microparticles are also less likely to cause stress concentrations and form cracks in the surfaces of the biocompatible scaffold.

Preferably, the scaffold bioactive glass component may be in the form of microspheres or microbeads.

Microparticles that are irregularly shaped provide a relatively rough surface topography, which is preferred by cells for adhesion and attachment. However, regularly shaped microparticles such as microspheres or microbeads, while still providing cell attachment and adhesion sites for the host tissue, have rounded, non-sharp edges, and are therefore less abrasive against the tissue. This means that the inflammatory response, if any, that will be produced by the use of microspheres or microbeads will be of a lower magnitude than the use of more irregularly shaped particles. The absence of sharp edges also drastically reduces the likelihood that stress concentrations and tearing will be caused in the surfaces of the biocompatible scaffold.

Spherical glass particles scatter light. Thus, when light is applied to the biocompatible scaffold comprising spherical glass particles, the light is scattered around and within the scaffold, thereby increasing the exposure of the biocompatible scaffold and biocompatible body to the applied light.

The microspheres or microbeads may be hollow.

The microparticles may be less than 30 µm in diameter.

The microparticles may be less than 10 µm in diameter.

The microparticles may be less than 1 µm in diameter.

The biocompatible scaffold may further comprises a scaffold water soluble filler component.

The scaffold water soluble filler component may fill at least a portion of the scaffold polymer component.

In one embodiment, the water soluble filler component can be used to insulate the attachment means from light and other environmental factors, thereby improving the shelf life of the attachment means. The filler component can be readily dissolved and removed from the scaffold polymer component prior to implantation by washing with water, or it can be left within the scaffold polymer component, to be dissolved *in vivo.*

The filler component may be configured to be used as a mechanism to deliver medicinal or wound healing agents to the tissue when dissolved in vivo. Alternatively, the filler component may have intrinsic medical or wound healing properties.

The water soluble filler component may be a sugar substance.

The sugar substance may be a form of toffee.

Advantageously, sugar, particularly in the form of toffee, stimulates wound healing when dissolved *in vivo.*

The water soluble filler component may be a polyvinyl alcohol substance.

The polyvinyl alcohol substance may be a polyvinyl alcohol gel.

The water soluble filler component may be a hydrogel substance.

The hydrogel substance may be a collagen hydrogel.

The biocompatible scaffold may further comprise a medicinal component.

Advantageously, the biocompatible scaffold is adapted to deliver a medicinal substance to the adjacent tissue in vivo.

The medicinal component may be impregnated within at least a portion of the biocompatible scaffold.

The medicinal substance may be an antibiotic substance, a wound healing agent, an anti-fungal agent, or a combination of these.

The biocompatible scaffold may further comprise a radio-opaque component.

Advantageously, the biocompatible scaffold is capable of being monitored by x-ray after implantation.

The radio-opaque component may be impregnated within at least a portion of the biocompatible scaffold.

The attachment means may be adapted to be an attachment of a medical device.

The attachment means may be configured to be removably attachable to a medical device.

The attachment means may be adapted for removal from the medical device.

Advantageously, the medical device can be resected and retrieved from the patient if required by resecting the attachment means. After long-term implantation, the attachment means is likely to have become well-integrated into the soft tissue of the patient but still cuttable by action of a surgical blade or scissors. Thus, potentially morbid or traumatic surgical removal of the attachment means from the host tissue can be avoided.

The attachment means may comprise one or more resectable soft tissue anchors.

Advantageously, removal of the attachment means from the medical device by cutting anchorage points or cutting the anchors themselves is simple and readily performed in a clinical or surgical setting.

The medical device may be a short term or temporary implant or a long term implant. For example, the medical device may be a pace maker, blood pump, hearing aid implant, bionic eye implant, deep brain stimulator, functional electrical stimulation device or neural interface.

According to a second aspect not in accordance with the invention, a system for attaching a medical device to tissue is provided, the system comprising:
at least one attachment means ; and
a light energy source.

The light energy source may be a low energy light source.

The light energy source may be a laser source.

The laser source may be a class 2M, class 3 such as a class 3R or class 3B laser or class 4 laser source.

Advantageously, these classes of laser are safe for use on a patient.

The laser source may be a green wavelength laser source.

Advantageously, green wavelength laser produces relatively low temperatures, and does not cause discomfort or pain to the patient when applied to the patient's skin.

According to a third aspect not in accordance with the invention, a medical device is provided, comprising:
a device body; and
at least one attachment means as described above attached to the device body.

The device body may be implantable.

The at least one attachment means is two attachment means, one attachment means being attached to each opposing side of the device body.

Advantageously, the attachment means secures the medical device to the patient to either side of the device body for a firm fixation.

The at least one attachment means may extend from a biocompatible cover which at least partly surrounds the device body.

The biocompatible cover is made from a polymer.

The at least one attachment means may be removable from the device body.

Advantageously, the medical device can be retrieved from the patient if required by simply resecting the soft tissue anchor. After long-term implantation, the attachment means is likely to have become well-integrated into the soft tissue of the patient and may be cut using a surgical blade or scissors. Thus, potentially surgical removal of the attachment means from the host tissue can be avoided.

The at least one attachment means may be in the form of an anchor retrievable by resection.

Removal of the attachment means from the medical device by cutting anchorage points or cutting the anchors themselves can be readily performed by for example, a physician in a clinical or surgical setting.

According to a fourth aspect not in accordance with the invention, a method of attaching the medical device to tissue is provided, the method comprising the steps of:
positioning the at least one attachment means within the tissue of a patient such that the biocompatible scaffold is in contact with the tissue; and
directing light energy to the biocompatible scaffold through the tissue to initiate crosslinking of the biocompatible scaffold to the tissue.

The light energy directed to the biocompatible scaffold may be continuous lightenergy.

The light energy may be pulsed light energy.

According to a fifth aspect not in accordance with the invention, a method of manufacturing the attachment means is provided, the method comprising the steps of:
- immersing a portion of the biocompatible scaffold in a biocompatible liquid polymer; and
- curing the biocompatible liquid polymer.

The method may further comprise the steps of:
- setting aside the product, being a first half of the attachment means,
- repeating the steps above to obtain a second half of the attachment means; and
- attaching the first half and the second half of the attachment means at their respective polymer surfaces.

The biocompatible liquid polymer may be a liquid silastomer.

The first half and second half one adhered.

According to a sixth aspect not in accordance with the invention, a method of manufacturing the attachment means, the method comprising the steps of:
- impregnating a top portion and a bottom portion of a biocompatible scaffold with a water soluble filler substance such that a middle portion of the biocompatible scaffold remains unfilled;
- injecting a biocompatible liquid polymer into the middle portion of the biocompatible scaffold; and
- curing the biocompatible liquid polymer.

The method may further comprise the step of:
- removing the water soluble filler substance by washing with water.

The biocompatible liquid polymer may be a liquid silicone rubber.

The water soluble filler may be a sugar substance.

The sugar substance may be a form of toffee.

The water soluble filler substance may be a polyvinyl alcohol substance.

The polyvinyl alcohol substance may be a polyvinyl alcohol gel.

The water soluble filler substance may be a hydrogel substance.

The hydrogel substance may be a collagen hydrogel.

The water soluble filler substance may be ice.

In an aspect in accordance with the invention, there is provided conformable cover for a medical device comprising:
a porous biocompatible scaffold;
a plurality of biocompatible filaments embedded within and fixed to the biocompatible scaffold, each of the biocompatible filaments comprising a shape-memory material and configured to transform from an extended configuration to a reduced configuration when the cover is heated to a predetermined temperature,
wherein, in use, upon covering a medical device with the cover and heating the cover to the predetermined temperature, the plurality of biocompatible filaments are configured within the scaffold to cause the cover to conform to an outer shape of the medical device.

At least one filament may comprise a plurality of filaments fixed to and embedded within the biocompatible scaffold in an arrangement configured to cause the scaffold to conform to an outer surface of a medical implant upon heating the cover to the predetermined temperature.

In another aspect not in accordance with the present invention, there is provided, an attachment device for connecting a medical device to biological tissue of a subject, the attachment device comprising:
a biocompatible scaffold;
at least one biocompatible filament embedded within and fixed to the biocompatible scaffold.

In yet another aspect not in accordance with the present invention, there is provided an attachment device for connecting a medical device to biological tissue of a subject, the attachment device comprising:.
a conformable cover for a medical device comprising:
a biocompatible scaffold;
at least one biocompatible filament fixed to and embedded within the biocompatible scaffold, the at least one biocompatible filament configured to transform from an extended shape to a reduced shape when heated to a predetermined temperature.

When a medical device is covered by the cover, in an embodiment, the at least one filament may comprise a plurality of filaments fixed to and embedded within the biocompatible scaffold in an arrangement configured to cause the scaffold to conform to an outer surface of a medical implant upon heating the cover to the predetermined temperature.

In an embodiment, the biocompatible scaffold may comprise a photoactive crosslinking agent for facilitating bonding of the cover to biological tissue. In an embodiment, the biocompatible scaffold may comprise a photoactive crosslinking agent for facilitating bonding of the cover to biological tissue.

The biocompatible scaffold may comprise polycaproplactone.

The biocompatible scaffold may be configured to facilitate soft tissue integration into the scaffold.

The biocompatible scaffold may be configured to facilitate hard or bony tissue integration into the scaffold.

The biocompatible scaffold may be configured to facilitate cartilage integration into the scaffold.

The biocompatible scaffold may have a heirarchical, porous interconnective structure.

The biocompatible scaffold may comprise a density gradient to facilitate integration of soft tissue, a transition to hard tissue and hard tissue within the scaffold.

The biocompatible scaffold may be formed by electrospinning.

The biocompatible filament may be formed by drawing.

The biocompatible filament may be formed by electrospinning.

The biocompatible filament may be made of a strain crystallized, shape memory polymer. The strain-crystallised, shape memory polymer may comprise polycaprolactone. The strain-crystallised, shape memory polymer may be drawn at a temperature within the range of 0 to 58 degrees Celsius and at a rate between 15 to 500 mm/second.

In another embodiment the strain-crystallised, shape memory polymer may be drawn at 20 degrees at a rate of 20mm/second.

The predetermined temperature may be within the range of 58 to 60 degrees.

The at least one biocompatible filament may include a photo-active material such that the filament is capable of crosslinking with biological tissue of a subject when light is directed onto the filament.

The photoactive crosslinking material may be chitosan.

Alternatively, the photoactive crosslinking material may be a synthetic polymer equivalent of chitosan.

The biocompatible scaffold may include a photo active dye.

The biocompatible scaffold may include a coating of comprising a photoactive coating comprising a photo-active crosslinking material and a photo-active dye.

At least one surface of the cover may include the photoactive coating.

The photo-active dye may be rose Bengal.

The photo-active dye may be methylene blue.

The photo-active dye may be indocyanine green.

The photo-active dye may comprise methylene blue and indocyanine green.

The photo-active dye may comprise a 50:50 ratio of rose Bengal and methylene blue.

In an embodiment, the biocompatible scaffold may be at least partially coated with the photoactive crosslinking agent and the photoactive dye.

The at least one biocompatible filament may be substantially elongate.

Alternatively, the biocompatible filament may have a helical shape.

In another embodiment, the biocompatible filament may comprise a light conductive, bioactive material.

The biocompatible filament may comprise a light conductive, bioactive material. For example, the biocompatible filament may be made of a bioactive glass such as 45S5 bioactive glass.

In an embodiment, the cover may comprise a layer of biocompatible scaffold. The layer may have a thickness, an inner surface and an outer surface. Each light conducting filament may extend from the outer surface through the entire thickness of the layer and to the inner surface of the layer.

In an embodiment of the device, the at least one biocompatible filament may comprise an arrangement of bioglass filaments extending through an entire thickness of the biocompatible scaffold. In an embodiment, the bioglass filaments may be arranged in a 2x2 matrix.

Each bioglass filament may extends through an entire length of the scaffold so as to direct conducted light onto an interface between the device and the biological tissue of a subject.

Ends of the bioglass filament may be located adjacent an interface between the device and tissue so as to direct conducted light onto an interface between the device and the biological tissue of a subject.

In an embodiment, the at least one filament may be oriented parallel to a surface of the cover within the biocompatible scaffold.

In another embodiment, the at least one filament perpendicularly to each of the inner surface and/or outer surface of the biocompatible scaffold of the cover.

In yet another embodiment, the at least one filament may be oriented at an angle to either the inner surface and/or the outer surface of the biocompatible scaffold layer of the cover.

The device may further include, at least one biocompatible conduit embedded within and/or fixed to the biocompatible scaffold.

The at least one biocompatible conduit may have an external diameter of 1.2 mm and an internal diameter of 0.6 mm.

The at least one biocompatible conduit may have a sealed end to retain a substance within the conduit. The substance may be a therapeutic substance such as a medicine.

The at least one biocompatible conduit may be semi-permeable to allow movement of cells, gases, and other biological material through the conduit.

The at least one biocompatible conduit may be made of semi-permeable material to allow medicine to slowly leach out of the semi-permeable material.

The cover may be in the form of a pouch configured to enclose a medical device, in use. The pouch may have an opening through which a medical device may be received, in use.

In another embodiment, the cover may be in the form of a sheet.

The attachment device may further include at least one biocompatible fastener for fastening one part of the biocompatible scaffold to another part of the biocompatible scaffold or another biocompatible scaffold to form an enclosure around the medical device, in use.

The biocompatible fastener may comprise the same biocompatible scaffold as the biocompatible scaffold of the cover.

In another embodiment, the biocompatible fastener may have a first part comprising a biocompatible scaffold with a surface texture including a plurality of loops.

In this embodiment, the biocompatible fastener has a second part comprising a surface texture including a plurality of hooks configured to engage with the plurality of loops of the first part.

The attachment device may further comprise at least one biocompatible anchor for anchoring the biocompatible scaffold to biological tissue of a subject, in use.

The biocompatible anchor may be made of biocompatible scaffold and comprise a photoactive crosslinking agent.

The biocompatible anchor may be made of biocompatible scaffold and comprise a photoactive crosslinking dye.

The biocompatible anchor may be capable of fusing to the biocompatible scaffold of the cover of the device when heated near a melting point of the biocompatible scaffold.

In another aspect of the attachment device not in accordance with the present invention, there is provided a method of making an attachment device for connecting a medical device to the tissue of a subject, comprises:
providing a biocompatible scaffold configured to facilitate biological tissue integration within the scaffold;
providing a plurality of biocompatible filaments, each filament comprising a strain crystallised, shape memory polymer;
embedding the plurality of biocompatible filaments within the biocompatible scaffold;
fusing each filament to the scaffold to fix each filament within the scaffold, and
selectively coating the biocompatible scaffold with a photoactive crosslinking agent and a photoactive dye.

The biocompatible filament may be in a helical shape.

Embedding the at least one biocompatible filament may comprise:
providing a helical wire substantially identical in shape and size to a filament of the at least one biocompatible filament;
the helical wire having a first end and a second end;
attaching one end of a helical biocompatible filament to the second end of the helical wire;
rotatably inserting the helical wire into the scaffold to create a helical channel to house the biocompatible filament within the scaffold;
removing the helical wire from the scaffold by continuing to rotate the helical wire through the scaffold until the path is filled with the biocompatible filament and the helical wire is out of the scaffold;
detaching the helical wire from the biocompatible filament.

In another embodiment, embedding the at least one biocompatible filament may comprise threading one or more of the at least one biocompatible filament through the eye of a needle, inserting the needle into the scaffold and moving the needle through the scaffold and then, removing the needle while leaving the filament remaining in the scaffold.

Fusing each filament to the scaffold to fix each filament within the scaffold may include solvent welding using acetone.

Fusing each filament to the scaffold to fix each filament within the scaffold may include solvent welding using a solution comprising acetone and polycaprolactone.

Fusing each filament to the scaffold to fix each filament within the scaffold may include gently spot welding each filament by contacting each filament using water at a temperature within the range of 60 degrees Celsius to 70 degrees Celsius.

Fusing each filament to the scaffold to fix each filament within the scaffold may include gently spot welding each filament by contacting each filament using water at a temperature within the range of 58 degrees Celsius to 72 degrees Celsius.

Selectively coating the biocompatible scaffold with a photoactive crosslinking agent and a photoactive dye may include coating the device with a coating solution including photoactive crosslinking dye and a photoactive dye.

Selectively coating the device may include dip coating at least part of the device by dipping the scaffold into a solution comprising photoactive crosslinking dye and a photoactive dye.

Selectively coating the device may include using a masking process to selectively coat at least part of the device.

The coating solution may comprise rose Bengal, chitosan and water.

The coating may comprise methylene blue and indocyanine green.

Selectively coated wherein the device is selectively coated such that the scaffold is sufficiently flexible to allow the scaffold to substantially conform to an outer shape of the device when the device is heated.

Selectively coating the scaffold may include immersing the biocompatible scaffold in a biocompatible solution and heating the device to a boiling point of the coating solution to remove excess liquid.

In an embodiment, of the method of making an attachment device for connecting a medical device to the tissue of a subject the attachment device may include a bioactive glass on an outer surface of or within the biocompatible scaffold of the cover. In this embodiment, the device may be selectively coated such that the bioactive glass is not masked by the coating and the bioactive glass is sufficiently exposed, in situ, to encourage infiltration of and adhesion of, cells and biological material to the scaffold and in turn, result in sufficiently strong bonding of the scaffold to the biological tissue of the subject.

In an embodiment, each light conductive, bioactive filament may extend through an entire thickness of the cover, between a first surface and an opposed surface of the cover such that one end of each filament is a light receiving node and the opposed end of the second filament is located adjacent an interface between biological tissue and the cover, in use.

In yet another embodiment not in accordance with the present invention, there is provided a method of making a device for connecting a medical device to tissue comprising:
providing a biocompatible scaffold;
providing an arrangement of cannulated needles, each needle having an internal channel;
inserting a biocompatible filament or biocompatible conduit in accordance with any
inserting the arrangement of cannulated needles at least partially into the scaffold to create voids within the scaffold to house each filament;
moving each cannulated relative to each filament to removing each cannulated needle from the scaffold; and
fixing the biocompatible filaments to the biocompatible scaffold.

The arrangement may comprise a 2-dimensional matrix of cannulated needles.

In an embodiment, the biocompatible filament may be made of bioglass.

The method may further include rounding the ends of the biocompatible filament.

The method may further include rounding the ends of the biocompatible filament using a laser cutter or a butane flame.

In yet another aspect not in accordance with the present invention, a method of connecting a medical device to biological tissue of a subject using an attachment device:
providing a medical device, the medical device having an outer shape;
providing an attachment device as summarized above;
positioning and securing the cover of the attachment device around at least part of the medical device such that the medical device will be retained within the cover after the cover conforms to the outer shape of the medical device;
applying sufficient heat to the cover of the device to cause the at least one biocompatible filament to change shape and cause the cover to substantially conform to the outer shape of the medical device;
positioning the attachment device including the medical device within the biological tissue of a subject such that the biocompatible scaffold is in contact with the biological tissue, and
directing light to the biocompatible scaffold to facilitate crosslinking of the biocompatible scaffold to the biological tissue.

The light may be white LED light.

The source of the LED light may have a wattage of 10 to 20 W.

Securing may include attaching one part of the biocompatible scaffold to another part of the biocompatible scaffold using electrocautery.

Applying sufficient heat may include immersing the cover in heated saline solution at the predetermined temperature.

In an embodiment, the cover of the attachment device may be in the form of a pouch and placing the attachment device around at least part of the medical device may include placing the medical device into the pocket of the device and sealing the pocket. In this embodiment, the cover may include applying sufficient heat or light to the cover of the device to cause the at least one biocompatible filament to change shape and cause the cover to substantially conform to the outer shape of the medical device can include immersing the closed pouch in a warm saline bath until the pouch has sufficiently conformed to the outer shape of the medical device. In this embodiment, the biocompatible scaffold of the cover of the attachment device does not include a photoactive crosslinking agent.

In another embodiment, the cover of the attachment device is in the form of a first pouch and placing the attachment device around at least part of the medical device may include placing the medical device into the first pouch of the attachment device and sealing the pouch. In this embodiment, the method may further include providing a second pouch made of biocompatible scaffold. The second pouch may include a photoactive crosslinking agent.

In another aspect not in accordance with the present invention, there is provided a method of connecting a medical device to biological tissue of a subject comprising:
providing a medical device, the medical device having an outer shape;
providing a first pouch comprising biocompatible scaffold for biological tissue integration;
providing an attachment device, wherein the conformable cover is in the form of a second pouch;
inserting the medical device within the first pouch and sealing the first pouch;
applying sufficient heat to the first pouch to cause the at least one biocompatible filament in the cover to contract and cause the cover to substantially conform to the outer shape of the medical device;
inserting the first pouch including the medical device into the second pouch;
positioning the second pouch adjacent the biological tissue of a subject such that the cover is in contact with the biological tissue at an interface, and
directing light to the interface between the biocompatible scaffold of the second pouch and the biological tissue of the second pouch to cause crosslinking of the second pouch to the biological tissue.

In this embodiment, the method may further include inserting saline into the second pouch using a syringe to cool the biological tissue while light is directed to the biocompatible scaffold. Advantageously, the saline filled second pouch helps conduct and guide light to an interface between the biological tissue and the second pouch.

Other aspects of the invention are also disclosed.

### Brief Description of the Drawings

Notwithstanding any other forms which may fall within the scope of the present invention, preferred embodiments of the present invention will now be described, by way of examples only, with reference to the accompanying drawings in which:
Fig 1 is a schematic perspective view of an implantable long term soft tissue engaging device having attachment means in the form of two attachment anchors in accordance with a preferred embodiment of the present invention, with laser light being applied to one of the attachment anchors; and
Fig 2 is an enlarged schematic cross-sectional view of one of the attachment anchors of Fig 1 disposed under the dermal layers of a patient.
Fig 3 is an enlarged schematic side view of one of the attachment anchors of Fig 1.
Fig 4 is an enlarged schematic cross-section of one of the attachment anchors of Fig 1, showing a plurality of macro-diameter canals.
Fig 5A depicts an alternative embodiment of the attachment device showing a conformable cover in the form of a pouch;
Fig 5B depicts the embodiment of the attachment device of Fig 5A after the conformable cover in the form of a pouch has conformed to an outer shape of a medical device;
Fig 6 illustrates a portion of a conformable cover in accordance with an embodiment of the attachment device;
Fig 7 illustrates an embodiment of the device and a method of using the device to connect a medical device to biological tissue;
Fig 8 illustrates an embodiment of the conformable cover provided as a sheet;
Fig 9 illustrates another embodiment of the conformable cover and a fastener;
Fig 10 illustrates anchors which can be provided as part of the attachment device in an embodiment;
Fig 12 illustrates a portion of the cover in accordance with an embodiment including light conducting filaments;
Fig 13 illustrates an attachment device in accordance with another embodiment and a method of using this device to connect a medical device to biological tissue.

### Description of Embodiments

It should be noted in the following description that like or the same reference numerals in different embodiments denote the same or similar features.

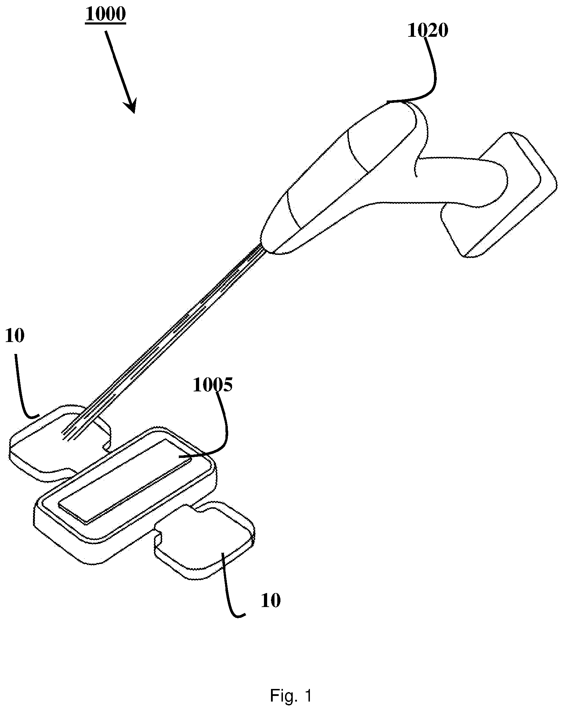

Referring to Fig 1, a device 1000 is shown, comprising a device body 1005 and attachment means 10. The attachment means 10 takes the form of two attachment anchors 10 attached to two opposing sides of the device body 1005 and is adapted to be implanted within the soft tissue of a patient to attach and substantially anchor the device 1000 to a patient.

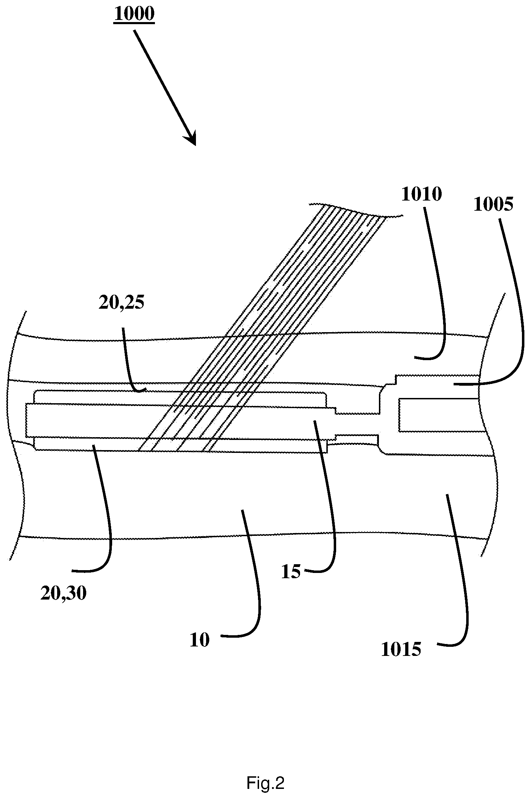

Fig 2 depicts one of the attachment anchors 10 disposed between the dermal layers 1010 of the patient's soft tissue and the vascularised tissue bed 1015 of the patient's soft tissue. Each attachment anchor 10 comprises a biocompatible body 15 and a biocompatible scaffold 20. In this embodiment, the biocompatible scaffold 20 surrounds the biocompatible body 15, such that at least a top surface 25 and a bottom surface 30 of the attachment anchor 10 is covered with the biocompatible scaffold 20. The top surface 25 and bottom surface 30 are the largest surfaces in contact with the dermal tissue layers 1010 and the vascularised tissue bed 1015 of the patient, respectively, when the attachment anchor 10 is implanted into a patient's soft tissue.

The attachment anchors 10 provide the fixation mechanism for the device 1000 to be anchored within the soft tissue of the patient. Once implanted, the biocompatible scaffold 20 provides a conformable structure into which soft tissue cells can infiltrate and vascularise. Once the cells and vasculature have sufficiently infiltrated into the biocompatible scaffold 20, a cellular-scaffold bio-adhesion and mechanical interlock is achieved, substantially fixing the attachment anchors 10 and the device 1000 to the patient. This biomechanical interlock has been shown to substantially anchor the attachment means 10 within the tissue within three to seven days of implantation.

In this embodiment, the biocompatible scaffold 20 comprises a scaffold polymer component that is bioresorbable, although in other embodiments, the scaffold polymer component is biostable. Thus, as the biocompatible scaffold 20 gradually resorbs within the in vivo environment, the infiltrated soft tissue grows to replace the biocompatible scaffold 20. When the biocompatible scaffold 20 has completely resorbed, the surrounding host tissue provides a relatively tight mechanical fixation. In this way, the biocompatible scaffold 20 can be described as mediating a stable, mechanical implant-tissue bridge. We note that in this embodiment, the biocompatible body 15 comprises a body polymer component that is biostable. In other embodiments, the body polymer component is bioresorble.

The more stably and firmly fixed an implant is within the host tissue, the lesser will be the inflammatory response of the host tissue, and thus the lower the likelihood of the host tissue developing a fibrous capsule or scar tissue. As the development of a thick fibrous capsule within the host tissue can potentially lead to clinical failure, use of the attachment anchors 10 for attaching medical devices to soft tissue substantially provides for the long- term clinical success of the medical device.

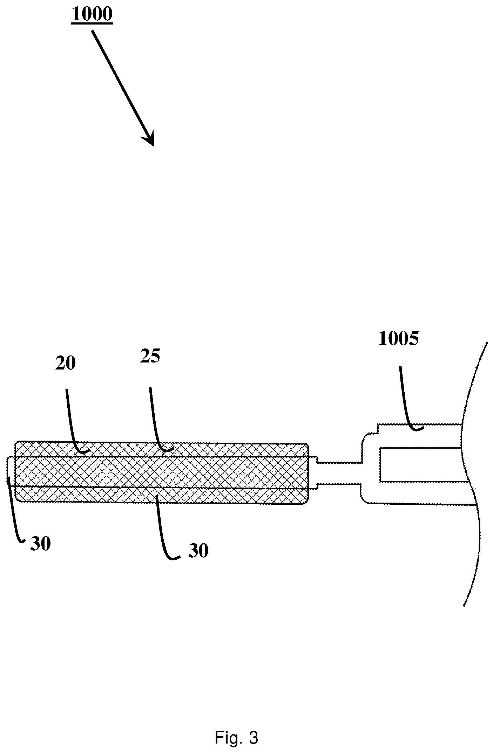

In each attachment anchor 10, the biocompatible scaffold 20 is impregnated within the biocompatible body 15 and extends through the entire thickness of the biocompatible body 15, such that the biocompatible scaffold 20 is a single unitary structure (see Fig 3). The host tissue is capable of replacing the biocompatible scaffold 20 by infiltrating into the small spaces within the biocompatible body 15 previously occupied by the biocompatible scaffold 20 as it gradually resorbs. When the biocompatible scaffold 20 is fully resorbed, the infiltration and vascularisation of tissue through the entire thickness of the biocompatible body 15 provides a strong, stable, long-term, mechanical implant-tissue interlock.

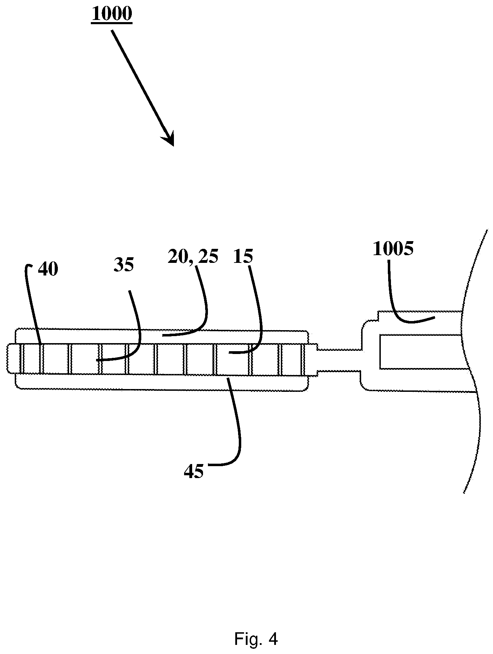

Referring to Fig 4, the body polymer component of the biocompatible body 15 comprises a plurality of macro-diameter canals 35 extending from a top surface 40 to a bottom surface 45. When implanted, the host tissue is able to infiltrate and vascularise through the canals and through the thickness of the biocompatible polymer body 15, providing an additional mechanism to improve the strength of the mechanical implant-tissue fixation.

Each of the macro-diameter canals 35 is substantially rounded in cross-section, such that no undue stress concentrations are created, which could otherwise weaken the biocompatible body 15. The macro-diameter canals 35 are between 1 mm to 15 mm in diameter, but preferably between 3 mm to 10 mm in diameter, being the ideal size range to encourage tissue infiltration and vascularisation.

In this embodiment, the biocompatible body 15 further comprises a body bioactive glass component that takes the form of a bioactive glass microparticle lining within the macro-diameter canals 35 (such as 45s5). Certain compositions of bioactive glass promote soft tissue growth and adhesion *in vivo.* The presence of bioactive glass within the canals 35 actively encourages soft tissue infiltration and adhesion within the canals 35 when implanted, and also prevents the formation of fibrous and scar tissue. In another embodiment, the body bioactive glass component takes the form of a bioactive glass microparticle coating on at least one surface of the body polymer component of the biocompatible body 15.

Microparticles, having relatively small diameters, do not create an excessively bumpy topography (in comparison with the topography larger particles would produce). Thus, when the microparticles are in contact with the host tissue, minimal irritation and abrasion is caused by the rubbing against the tissue. Microparticles are also less likely to cause stress concentrations and form cracks in the surface of the biocompatible body 15. More specifically, the microparticles are preferably in the form of microspheres or microbeads.

Microparticles that are irregularly shaped provide a relatively rough surface topography, which is preferred by cells for adhesion and attachment. However, regularly shaped microparticles such as microspheres or microbeads, while still providing cell attachment and adhesion sites for the host tissue, have rounded, non-sharp edges, and are therefore, less abrasive against the tissue. This means that the inflammatory response, if any, that will be produced by the use of microspheres or microbeads will be of a lower magnitude than the use of more irregularly shaped particles. The absence of sharp edges also drastically reduces the likelihood that stress concentrations and tearing will be caused in the surface of the biocompatible body 15.

Furthermore, spherical glass particles scatter light. As the canals extend from the top surface 40 to the bottom surface 45 of the biocompatible body 15, the spherical bioactive glass microparticles helps light applied at the top surface 40 travel through the biocompatible body 15 to the bottom surface 45. In another embodiment, the microspheres or microbeads are hollow. The microparticles should be less than 30 µm in diameter, but ideally less than 1 µm in diameter.

In this embodiment, the body polymer component of the biocompatible body 15 is made of a biocompatible elastomer, specifically a silastic material. In other embodiments, the body polymer component is made of a polyurethane material or a polycarbonate material.

The body polymer component of the biocompatible body 15 has a high transparency. In this embodiment, the application of light to the biocompatible scaffold 20 forms a step in the method of causing the attachment anchors 10 to attach the device 1000 to tissue. The transparency of the body polymer component of the biocompatible body 15 allows light applied to the biocompatible scaffold 20 covering the top surface 40 of the biocompatible body 15 to reach the biocompatible scaffold 20 covering the bottom surface 45 of the biocompatible body 15. Thus, even underlying portions of the biocompatible scaffold 20 can be exposed to the applied light.

The biocompatible scaffold 20 further comprises a network of interconnective micropores, providing the necessary pathways through which cellular integration, vascularisation, oxygenation and toxin drainage can occur. The network of interconnective micropores ideally has a non-repeating, randomised structure, through which blood vessels can more naturally navigate. Repeating structures, though still capable of being used as a cellular scaffold, require blood vessels to take more tortuous paths.

The network of interconnective micropores comprises a plurality of relatively small micropores located within a plurality of relatively large micropores, such that the small micropores provide adhesion sites for cells and the larger micropores allows vascularisation, toxin drainage and tissue growth. The relatively large micropores should be 60 µm to 500 µm in diameter. The relatively small micropores should be 5 µm to 15 µm in diameter. The biocompatible scaffold 20 should be highly porous, such as greater than or equal to 90vol% porosity or each greater than 95vol% porosity.

Having a high porosity scaffold 20 allows cells and tissue to infiltrate and navigate through with very little obstruction, while still providing a framework on which the tissues can anchor and be supported. A highly porous biocompatible scaffold 20 is also mechanically advantageous, as it is soft, compressible and extensible, avoiding the production of stress concentration which may cause damage to the surrounding tissue in use. Being compressible and extensible also allows the biocompatible scaffold 20 to conform intimately to the tissue interface to ensure reliable contact and adhesion. These factors reduce irritation, the likelihood of infection and resultant inflammatory response at the implant-tissue interface, which in turn reduces the likelihood that a thick fibrous, fluid filled capsule will be formed to isolate the attachment means 10 from the surrounding tissue and cause loosening.

The biocompatible scaffold 20 can be in the form of a non-woven pile, a felt structure, a 3D fabric, or a 3D mesh or sponge, or take any other suitable form. The scaffold polymer component of the biocompatible scaffold 20 is preferably made of a medium to high molecular weight aliphatic polyester thermoplastic (such as polycaprolactone). In another embodiment, the scaffold polymer component is made of a polypropylene. In other embodiments, the scaffold polymer component may comprise one selected from the following list: biodegradeable polyurethane or polylactic acid (PLA) or poly-L/D-lactide (PLDLA) or polyvinyl alcohol (PVA).

In this embodiment, a bonding property of the biocompatible scaffold 20 is capable of being activated in use. Specifically, the biocompatible scaffold 20 comprises a scaffold crosslinking component capable of crosslinking to tissue in use when activated by light energy. Fig 1 shows a light energy source 1020 being used to apply light energy on the biocompatible scaffold 20. Fig 2 shows the light energy being applied to the already implanted biocompatible scaffold 20 through the patient's dermal layers 1010. The scaffold crosslinking component crosslinks to tissue within one to two minutes of light energy application. The light energy can be applied either continuously or pulsatingly, depending on the intensity of the light energy. In another embodiment, the scaffold crosslinking component is activable by heat, either applied to the biocompatible scaffold 20 by external means, or applied by the patient's own body.

The light- or heat-activated scaffold-tissue crosslinking provides a mechanism by which the biocompatible scaffold 20 can be initially adhered to the surrounding tissue upon implantation. As the cellular bio-mechanical attachment described above takes around three to seven days before the attachment means 10 is firmly anchored, this initial adherence substantially immobilises the attachment means 10 within the host tissue at an early time point (within minutes), and facilitates the slower, longer term fixation of tissue infiltration into the biocompatible scaffold 20. Body heat activated crosslinking is advantageous as the bonding would spontaneously occur when the attachment anchors 10 is implanted into the patient.

In this embodiment, the scaffold crosslinking component is made of a photoactive agent, such as a chitosan film or a synthetic polymer equivalent. Chitosan comprises an initiator that frees up chemical bonds upon application of light energy. The liberated chemical bonds rapidly crosslink to the collagen component of cells. Chitosan film is also capable of forming a strong mechanical anchor to the surfaces of the biocompatible scaffold 20. In another embodiment, the scaffold crosslinking component is made of a heat-active agent, such as a polyvinyl alcohol gel, which crosslinks to tissue when heated to body temperature.

In this embodiment, the light energy source is a low energy light source such as a class 2M green wavelength laser emitter. Class 2M laser is safe for use on humans, and green wavelength laser produces relatively low temperatures that will not cause discomfort or pain to the patient when applied to the patient's skin. Fig 1 depicts a handheld laser emitter 1020.

In this embodiment, the biocompatible scaffold 20 further comprises a scaffold bioactive glass component in the form of microparticles either coated on or impregnated within the scaffold polymer component. Certain compositions of bioactive glass promote soft tissue growth and adhesion *in vivo.* The presence of bioactive glass within the biocompatible scaffold 20 will actively encourage soft tissue infiltration into and adhesion to the biocompatible scaffold. The adhesion of the tissue to the scaffold *in vivo* prevents or substantially reduces the formation of fibrous and scar tissue.

Microparticles, having relatively small diameters, when present on the surfaces of the biocompatible scaffold 20, do not create an excessively bumpy topography (in comparison with the topography larger particles would produce). Thus, when bioactive glass component is in contact with the host tissue, minimal irritation and abrasion is caused by the rubbing of the surface against the tissue. Microparticles are also less likely to cause stress concentrations and form cracks in the surfaces of the biocompatible scaffold. In this embodiment, the microparticles are less than 30 µm in diameter, or more restrictively, less than 1 µm in diameter.

In this embodiment, the microparticles of the scaffold bioactive glass component are in the form of microspheres or microbeads. Microparticles that are irregularly shaped provide a relatively rough surface topography, which is preferred by cells for adhesion and attachment. However, regularly shaped microparticles such as microspheres or microbeads, while still providing cell attachment and adhesion sites for the host tissue, have rounded, non-sharp edges, and are therefore less abrasive against the tissue. This means that the inflammatory response, if any, that will be produced by the use of microspheres or microbeads will be of a lower magnitude than the use of more irregularly shaped particles. The absence of sharp edges also drastically reduces the likelihood that stress concentrations and tearing will be caused in the surfaces of the biocompatible scaffold 20.

Furthermore, spherical glass particles scatter light. Thus, when light is applied to the biocompatible scaffold 20 comprising spherical glass particles, the light is scattered around and within the biocompatible scaffold 20, thereby increasing the exposure of the biocompatible scaffold 20 to the applied light.

In one embodiment, the biocompatible scaffold 20 further comprises a scaffold water soluble filler component that fills at least a portion of the scaffold polymer component.

The water soluble filler component can be adapted for use for a range of purposes, depending on the substance used. The water soluble filler component can be used to insulate the attachment anchors 10 from light and other environmental factors, thereby improving the shelf life of the attachment anchors 10. For this use, the filler component can be readily dissolved and removed from the scaffold polymer component prior to implantation by washing with water, or it can be left within the scaffold polymer component, to be dissolved *in vivo.* The filler component can also be used as a mechanism to deliver medicinal or wound healing agents to the tissue when dissolved *in vivo.* Alternatively, the filler component could have intrinsic medical or wound healing properties.

In one embodiment, the water soluble filler component is a sugar substance such as toffee. Sugar, particularly in the form of toffee, has been shown to stimulate wound healing when dissolved *in vivo.* In another embodiment, the water soluble filler component is a polyvinyl alcohol substance, such as a polyvinyl alcohol gel. In another embodiment, the water soluble filler component is a hydrogel substance, such as a collagen hydrogel.

In another embodiment, the biocompatible scaffold 20 further comprises a medicinal component impregnated within it, such that the biocompatible scaffold 20 delivers a medicinal substance to the adjacent tissue when implanted, improving the clinical success of the implant. The medicinal substance could be an antibiotic substance, a wound healing agent, an anti-fungal agent, or a combination of these.

In another embodiment, the biocompatible scaffold 20 further comprises a radio-opaque component impregnated within it, such that the biocompatible scaffold 20 is capable of being monitored by x-ray after implantation. The radio-opaque component is biocompatible and bioresorbable.

In this embodiment, the attachment anchors 10 are easily removed by cutting anchorage points and graft anchors themselves, when required, from the device 1000. Thus, the device is easily resected and retrieved from the patient (e.g. if replacement is required). After long-term implantation, the attachment means 10 is likely to have become well-integrated into the soft tissue of the patient. Being easily removable allows potentially invasive surgical removal of the attachment means 10 from the host tissue to be avoided.

In this embodiment, the biocompatible body 20 is attached, adhered to or integral with the device body 1005.

In another embodiment, the device body 1005 comprises a biocompatible cover made of a biocompatible polymer. In one embodiment, the attachment anchors 10 extends to, is attached to or adhered to the biocompatible cover. In another embodiment, the body polymer component of the biocompatible body 15 is integral with the biocompatible cover.

### Method

The figures disclose a method of attaching the device 1000 to tissue, in which the two attachment anchors 10 are positioned within the soft tissue of a patient, in between the dermal layers 1010 and the vascularised tissue bed 1015 of the patient's soft tissue. In this position, the biocompatible scaffold 20 covering the biocompatible body 15 is in direct contact with the host tissue of the patient. When the attachment anchors 10 are in position, light energy is directed the patient's skin at the implant site. The light energy received by the biocompatible scaffold 20 through the patient's skin is sufficient to initiate crosslinking of the scaffold crosslinking component of the biocompatible scaffold 20 to the tissue. Depending on the intensity of the light energy, the light energy could be applied either continuously or pulsatingly, to initiate the crosslinking.

### Infiltration gravity cast method

One method of manufacturing the attachment means 10 is infiltration gravity casting. This method comprises the steps of:
- immersing a portion of the biocompatible scaffold in a biocompatible liquid polymer; and
- curing the biocompatible liquid polymer.

The biocompatible liquid polymer, when cured, forms the body polymer component of the biocompatible body. The steps described in the previous paragraph yield an attachment means comprising a biocompatible scaffold that covers only one surface of the biocompatible body. Nevertheless, the biocompatible scaffold is substantially impregnated within the body polymer component of the biocompatible body. The attachment means yielded from this process shall be referred to as a 'single sided scaffold' attachment means.

To yield an attachment means comprising a biocompatible scaffold that covers two opposing surfaces of the biocompatible body 15 (as pictured in Figs 2 to 4), the following steps are taken:
- set aside the first 'single sided scaffold' attachment means,
- obtain a second 'single sided scaffold' attachment means by repeating the two steps described above;
- attach the first and second 'single sided scaffold' attachment means together at their respective polymer surfaces using adhesive or otherwise.

In this embodiment, the biocompatible liquid polymer is a liquid silastomer, and the adhesive is a silicone adhesive.

### Injection moulding and temporary fillers

Another method of manufacturing the attachment anchors is injection moulding. The method comprises the steps of:
- impregnating a top portion and a bottom portion of a biocompatible scaffold with a water soluble filler substance such that a middle portion of the biocompatible scaffold remains unfilled;
- injecting a biocompatible liquid polymer into the middle portion of the biocompatible scaffold; and
- curing the biocompatible liquid polymer.

If the water soluble filler substance is intended to be dissolved or resorbed *in vivo,* it can be left within the biocompatible scaffold. Alternatively, the method comprises the further step of:
- removing the water soluble filler substance by washing with water.
In this embodiment, the biocompatible liquid polymer is a liquid silicone rubber. The water soluble filler substance can be a sugar substance, such as a toffee, a polyvinyl alcohol substance, such as a polyvinyl alcohol gel, or a hydrogel substance, such as a collagen hydrogel, or ice.

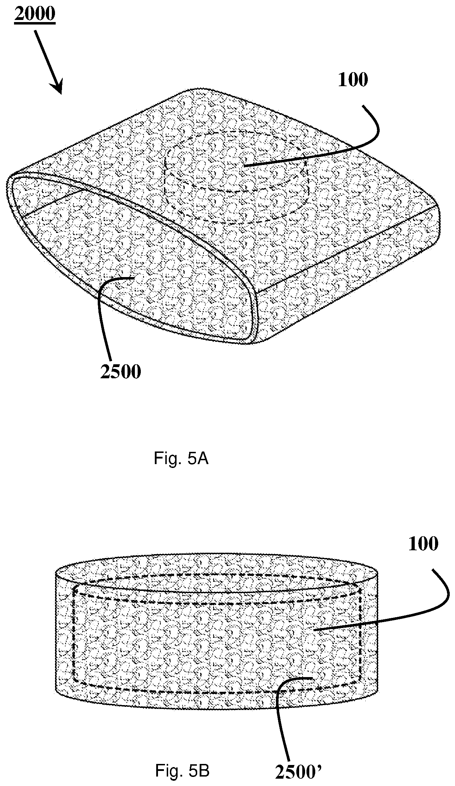

Figures 5A and 5B depict an embodiment of the attachment device 2000 including a conformable cover 2500 is provided. In this embodiment, the attachment device includes a conformable cover made of a biocompatible scaffold-filament composite. The cover 2500 includes a photo-active crosslinking agent for facilitating attachment of the device to biological tissue when light is directed to the cover 2500. The cover 2500 comprises a biocompatible scaffold and a plurality of biocompatible filaments (not shown) which are embedded within and fixed to the biocompatible scaffold. Each of the biocompatible filaments are made of strain crystallised shape memory polymer and are in an extended configuration of the polymer. Each of the plurality of biocompatible filaments are configured to transform from the elongated or extended or strain crystallised configuration, to a reduced or relaxed configuration of the shape memory polymer when the cover 2500 is heated to a predetermined temperature. The predetermined temperature is within the range of 57 degrees Celsius to 62 degrees Celsius.

When a medical device 100 is covered by the cover 2500, the plurality of filaments are in an arrangement configured to cause the scaffold to substantially conform to an outer surface of a medical implant 100 upon heating the cover to the predetermined temperature as shown in Figure 5B. The conformed cover is indicated by reference numeral 2500'. The cover 2500 is configured to conform to an outer shape of the medical device 100 in a manner similar to "shrink-wrapping".

In this embodiment, the cover 2500 includes a photo active crosslinking agent to enable attachment of the cover 2500 to biological tissue when for example, light is directed to a scaffold-tissue interface or the scaffold-tissue interface is sufficiently illuminated to cause crosslinking of the biological tissue to the cover and therefore, strong bonding between the biological tissue and the biocompatible scaffold.

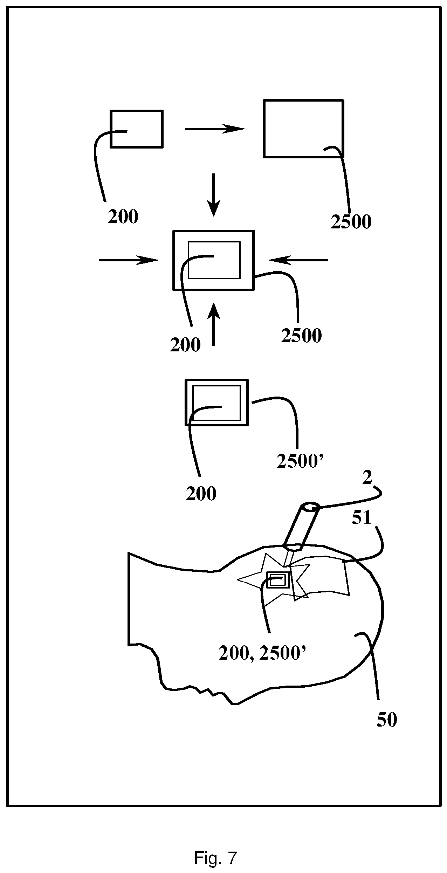

Figures 5A, 5B and 7 illustrate an embodiment where the cover 2500 is in the form of a pouch. Figure 5A illustrates a pouch 2500 having an opening to receive an implant or medical device 100 to be implanted within the biological tissue of a subject or attached to the biological tissue of the subject. The medical device 100 is positioned within the pouch. As mentioned above, Figure 5B illustrates the pouch after it has been sealed and sufficiently heated to a predetermined temperature by, for example immersing the sealed pouch including the medical device 100 in saline at the predetermined temperature, causing the pouch to conform to the outer shape of the implant 100.

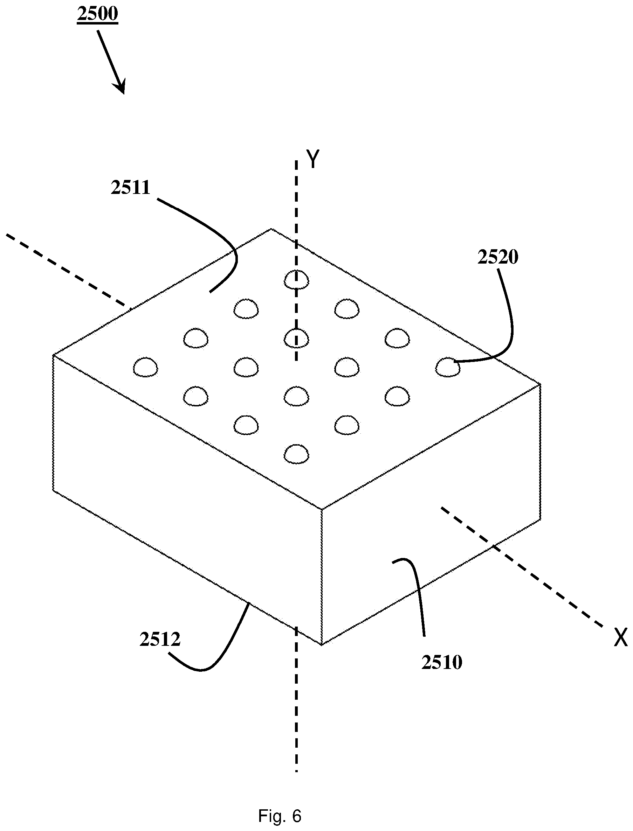

Figure 6 shows a portion of the cover including the biocompatible scaffold 2510 which substantially comprises polycaprolactone (PCL). The scaffold 2510 has a hierarchical, interconnected porous structure. The biocompatible scaffold 2510 comprises a combination of fibrous and crystalline PCL.

The biocompatible scaffold 2510 is configured to be bioactive and biomimetic to provide an environment conducive to biological tissue integration within the scaffold 2510. For example, the scaffold 2510 can have mechano-transductive properties to encourage cell proliferation and differentiation. It is envisaged that in other embodiments, various types of biocompatible scaffolds 2510 can be provided that are configured for various applications. For example, the scaffold 2510 can be configured to be conducive to either bony or soft tissue growth, or have a different a hierarchical, interconnected porous structure having different percentages of porosity e.g. within the range of 10% to 98%. It is envisaged that the scaffold 2510 can be made using a number of processes such as electrospinning or batch coagulation or melt spinning or templating or a workable combination of these methods.

In another embodiment, the scaffold 2510 can also be a graded scaffold (not shown) having a density gradient within the scaffold. This will enable soft tissue to infiltrate and integrate within the lower density parts of the scaffold and for example, bony tissue to integrate within the higher density parts of the scaffold.

Figure 6 shows a portion of the cover 2500 which includes a 2-dimensional array of elongate filaments 2520 embedded within the biocompatible scaffold 2500, each which linearly extend from an inner side of the cover 2500 that is in contact with the implant, in use, through the thickness of the biocompatible scaffold 2510 and to an opposed outer side of the cover 2500.

The filament 2520 is made of a shape memory polymer that has been strain crystallized. The strain crystallised filament 2520 is in an elongate and relatively straight configuration. By contrast, the original or reduced configuration of the polymer (not shown) has a shorter overall length. Upon heating of the strain-crystallized filament 2520 to the predetermined temperature, it reverts to its original, reduced shape. Advantageously, when a plurality of filaments 2520 is embedded in the scaffold 2510 and fixed to the scaffold 2510, heating of the cover at the predetermined temperature causes each filament to simultaneously transform from the elongated configuration to the original or reduced configuration. As each filament 2520 contracts, the scaffold is tensed and pulled tightly around the medical device.

In the embodiment of figure 6, each filament 2520 is a composite of a biocompatible polymer and a biocompatible glass. The major constituent of each filament 2520 is polycaprolactone. Each filament 2520 has approximately 20wt% bioactive glass. Each filament 2520 also includes a trace amount of carbohydrate (>1%, containing sucrose, glucose, fructose). In other embodiments, the bioactive glass composition can be within the range of 0 to 30wt%.

Strain crystallised filaments 2520 can be formed by a drawing process suitable for drawing plastics into threads of filaments.

In another embodiment, strain crystallised filaments 2520 can be formed by an electrospinning process.

A method of forming each of the strain crystallised filaments 2520 using a drawing process is now described. Precursor material for the filaments is formed from the biocompatible scaffold which includes polycaprolactone (linear aliphatic polyester) with a molecular weight of 84,000 g/mol and 1.15 g/cm3. In this embodiment the biocompatible scaffold does not include bioglass.

The scaffold-powder mixture is then heated to form a molten mixture. Bioglass can be added to the molten mixture at this stage. The diameter of each grain of powder of bioglass is within the range of 10 to 15 microns. The molten mixture is masticated for 20 seconds using a roller to eliminate any agglomerates and ensure even powder distribution within the mixture. The mixture is plastically formed or extruded while molten into a rod, preferably with an aspect ratio 4:1. This improves the consistency of drawing, minimizes the risk of polymer tear or fracture, and slippage of the drawing apparatus. In other embodiments, the precursor can be in the form of a hollow cylinder or a pyramidal or bone shape. In other embodiments, the aspect ratio can be within the range of 3:1 to 10:1) then allowed to cool to standard lab conditions (22 degrees Celsius).

The rod has two ends. The rod is creased or gentle crimped in its centre using a clamp.

One end of the rod is clamped in a holder and the other end is engaged with a rotating spool. The spool is rotated at for example, 20 mm/second at a temperature between 0 to 58 degrees Celsius which draws a relatively thin, strain-crystallised polycaprolactone filament from the rod. In other embodiments the spool can be rotated within the range of 10 to 500mm/second to draw filaments with different diameters.

The linear aliphatic polyester composite is typically 55-60% crystallinity in initial processing steps and 30-45% with subsequent melting-cooling steps, with no flow induced or strain-induced crystallization.

The crystallinity change induced by cold drawing leads to a measurable crystallinity increase to around 80% (or in the range of 70-90%).

Strain crystallization of each filament via mechanical drawing (or straining) occurs at temperatures below melting range of the polycaprolactone i.e. less than 60 degrees Celsius to induce molecular alignment (crystallization) of the polymeric chains in the direction of drawing.

Drawing at temperatures greater than 59 degrees Celsius leads to polymer melt forming in the drawn specimen then cooling into a semi-crystalline polymer form without strain-induced crystallization.

Strain crystallization leads to realignment of the polymer chains such that most primary covalent bonding (along chains) align with the draw direction, while most of the secondary bonding (weak) is perpendicular to draw direction. This results in greater strength, resilience, and minimizes deleterious creep in the direction of crystallization. The alignment of chains and higher concentration and intimacy for secondary bonding along the chains reduces chain slippage that contributes to creep over time.

The material composition, thermo-mechanical history, and its temperature compared to the melting range will lead to the drawing (strain) rate inducing a varying degree of crystallinity, and respective reduction in section thickness of the drawn specimen.

Residual stress is stored in the strain-crystallized filament. This can be returned as force, displacement, and actuation work when the filament or drawn specimen is warmed close 57-60 deg C. Hence, when the cover housing a medical device is placed in saline at approximately 59 degrees, the filaments contract and force the scaffold to mold around the medical device. With relatively high temperatures (i.e. temperature greater than 60deg C) and efficient heat transfer (e.g. as provided by immersing in warm saline) the contraction rate of the specimen can increase to 50mm/sec.

In the embodiment shown in Figure 6, the diameter of each filament 2520 is 20 micrometers. In other embodiments, the diameter of the filament can be within the range of 1 to 50 micrometres. In other embodiments, the diameter of the filament can be less than 1 micrometer or greater than 50 micrometres.

In the illustrated embodiment, each of the filaments 2520 are substantially elongate and straight. In another embodiment, the filaments 2520 can be crimped. In yet another embodiment, the filaments can have a helical shape (not shown). In another embodiment, the plurality of filaments can include a combination of straight, crimped and/or helical filaments.

In the embodiment shown in Figure 6, the portion of the cover 2500 includes a 4x4 matrix arrangement of straight filaments extending from one side 2511 of the scaffold 2510 to the other side 2512 of the scaffold. In this embodiment, each filament reinforces the strength of the cover in the axial direction of the filament (average axial direction is indicated by the Y axis) i.e. forming a reinforced composite. In this way, the orientation, diameter and/or shape of the filament 2520 can be selected to selectively reinforce the biocompatible scaffold 2510.

In the embodiment of Figure 6, the PCL filaments 2520 can reduce in length by up to 90% of the length of filament 2520 in strain crystallised form.

The filament 2520 will act to contract and therefore, pull adjacent parts of the biocompatible scaffold 2510 in a direction aligned with the axis of the elongated filament.

For example, a vertically oriented elongated filament extending through the thickness of the cover will cause to reduce the thickness of the cover. By contrast, a horizontally oriented elongated filament will act to reduce a length of the cover in the direction of the axis of the filament (such as along the X-axis as shown).

It is envisaged that in other embodiments, filaments can be embedded in various directions within the biocompatible scaffold to cause the cover to contract or conform to the shape of the implant in a particular way.

It is also envisaged that the number of filaments, material, diameter and orientation type to suit application to adjust mechanical properties of the cover in its unconfirmed and conformed shapes.

In the embodiment of Figure 6, the filaments increase the strength of the composite in the Y-direction or axial direction of the filaments is 800% higher than the strength of the scaffold without any embedded filaments. In figure 6, the porosity of the scaffold is 95% when the portion of scaffold was tensed in a tensile testing machine. However, there is negligible strength increase in the lateral or x- direction of the portion of the cover compared to the biocompatible scaffold without the embedded filaments.

In another embodiment (not shown) the filament can comprise a polycaprolactone core which can then be coated with bioactive glass to form a macro-composite. This can be achieved by dipping the filament for 5-10 seconds in an acetone-bioactive glass suspension (10 vol%), or in a PCL-acetone-bioactive glass suspension containing 10 wt% PCL (in the range of 1-20 wt% PCL) and 10 wt% bioactive glass (or in the range of (0-20 wt% bioactive glass).

In another embodiment (not shown), rose bengal and methylene blue dye can be added to the precursor material comprising polycaprolactone before the filaments are drawn. The biocompatible scaffold is coated with an aqueous mixture containing an even mixture of rose bengal (50vol%), methylene blue (50vol%) dye then dried before being warmed (60 deg C then masticated). Advantageously, each of the filaments in this embodiment are capable of photoactive crosslinking i.e. the filaments will be configured to crosslink with biological tissue when light is directed at the interface between the scaffold and/or filaments and biological tissue. Another use of the attachment device in this embodiment is as suture/filament that then fuses to tissue by shining light along the filament.

After filaments are embedded within the scaffold, they can be attached or fused to scaffold by solvent welding using an acetone solution.

In an example, the filaments can be fused to the scaffold by solvent welding using a solution comprising acetone and 10wt% polycaprolactone. Advantageously, heat is not applied using solvent welding so the strain crystallized filaments are less likely to revert to their natural state during the fusing process.

Acetone can also roughen the surface texture of the struts within the biocompatible scaffold and/or the filament which will encourage cell adhesion.

In another example, fusing each filament to the scaffold to fix each filament within the scaffold may include gently spot welding each filament by contacting each filament using water at a temperature within the range of 58 degrees Celsius to 70 degrees Celsius. This is useful for filaments which are not strain-crystallised.

After the filaments 2520 are attached to the scaffold 2510 forming a scaffold-filament composite, the scaffold-filament composite is coated with a photoactive coating. The scaffold-filament composite may be selectively coated. The scaffold-filament is selectively coated such that the cover is sufficiently flexible to allow the scaffold to substantially conform to an outer shape of the device when the device is heated and also be able to be crosslinked to biological tissue. Crosslinking forms strong carbon bonds with biological tissue and tends to stiffen the cover.

Furthermore, the cover 2500 may include bioactive glass on an outer surface of or within the biocompatible scaffold of the cover, selectively coating the scaffold-filament composite will not cover the bioactive glass and render it functionally obsolete. In this embodiment, the device may be selectively coated such that the bioactive glass is not masked by the coating and the bioactive glass is sufficiently exposed, in situ, to encourage infiltration of and adhesion of, cells and biological material to the scaffold and in turn, result in sufficiently strong bonding of the scaffold to the biological tissue of the subject.

In an embodiment, coating the biocompatible scaffold with a photoactive crosslinking agent and a photoactive dye may include coating the device with a coating solution including photoactive crosslinking dye and a photoactive dye.

In another embodiment, selectively coating the device may include dip coating at least part of the device by dipping the scaffold into a solution comprising photoactive crosslinking dye and a photoactive dye.

In yet another embodiment, selectively coating the device may include using a masking process to selectively coat at least part of the device.

The coating solution can comprise rose Bengal, chitosan, methylene blue and water.

In another embodiment, the coating can comprise methylene blue and indocyanine green or a combination of polycaprolactone, chitosan and acetic acid and water.

Advantageously, when white LED light is directed to the cover, a greater spectrum of wavelengths of visible can be used to cause crosslinking when methylene blue and indocyanine green are both used compared to when either methylene blue or indocyanine green is used.

In another embodiment the coating solution can include rose Bengal and methylene blue. This will again increase the efficiency of the cross linking as different wavelengths and therefore more of the directed light can be harnessed for crosslinking.

The device 2000 can be selectively coated such that the scaffold is sufficiently flexible to allow the scaffold to substantially conform to an outer shape of the device when the device is heated.

Selectively coating the scaffold may include immersing the biocompatible scaffold in a biocompatible solution and heating the device to a boiling point of the coating solution to remove excess liquid.

An embodiment of coating the scaffold-filament composite with a bioactive solution is now described.

1.7% w/v of medium molecular weight chitosan is dissolved in deionised water containing 2% v/v acetic acid (in other embodiments, in the range of 1-5% v/v) and 0.01%w/v Rose Bengal dye (or in other embodiments within the range of 0.005-0.1%w/v) and Methylene Blue dye 0.01%w/v (or in other embodiments, within the range of 0.000-1% w/v) . The target pH of the solution should be 3.5, potentially in the range of pH 3-6.

The photodynamic solution was rigorously stirred (protected from exposure to light at all times using) for 14 days (alternatively in the range of 0.1 to 14 days) at an ambient temperature of 25 degrees Celsius to dissolve the dye.

Stirring time can be reduced by using simultaneous ultrasound application to the mixture and a number (2-10) of short microwave exposure steps (5-25 seconds 1200 watt) to cause gentle warming (26-35 degrees Celsius) followed by cooling. Alternatively, vortex mixers or spex-milling process (high-energy ball mill) can be utilized to reduce stirring time from 14 days to hours.

These steps and subsequent ones, including scaffold-filament composite handling, transport, sterilization, storage must eliminate any exposure to light to maintain activity.

The photoactive dye is then centrifuged at 3270g (or in the range of 3000-24000g) for 1h (or in the range of 5-60 mins) at room temperature to separate out any impurities.

The aqueous dye solution is balanced (by drying or adding water) to ensure 0.1vol % photodynamic dye solution or in the range of 0.01-10 vol%

The scaffold-filament composite is then soaked in the solution. In particular, the scaffold-filament composite is palpitated, soaked and vacuumed to ensure thorough infusion and removal of entrapped air bubbles.

The scaffold-filament is air dried for 7 days or alternatively dried in a in a drier for 24 hours (or in other embodiments, within the range of 5-48 hours) with setpoint at 55 deg C (or in other embodiments, within the range of 35-57 deg C).

White LED light is directed to cause crosslinking liberates oxygen. The cover includes a coating comprising a cross-linking polymer component and at least one photoactive dye. The photoactive dye can be methylene blue, indocyanine green, fluorescein and/or rose Bengal in a solution with a crosslinking polymer component. Using a combination of photoactive dyes improves the efficiency of the biocompatible scaffold-filament composite in absorbing and reacting with white LED light as the dyes are sensitive to and so can harness different bands of wavelengths of light to cause crosslinking.

Figure 7 depicts a process of implanting an implant such as a cochlear implant 200 using the attachment device 2000.

The cover 2500 is provided as pouch. The cochlear implant 200 is placed inside the pouch and the pouch is sealed. The pouch can be sealed by fusing the parts of the scaffold to seal closed the mouth of the pouch using solvent welding or by selectively heating open edges of the pouch near a melting temperature of the scaffold and pressing them together to sealingly close the mouth of the pouch.

The closed pouch is then placed in a saline bath at approximately the predetermined temperature. The predetermined temperature is approximately 57 degrees Celsius. Immersing in a saline bath allows the pouch with the medical device to be evenly heated by the saline. The pouch then conforms to the outer shape of the medical device 200.

An incision is made in the head of the patient 50 adjacent the ear, by a surgeon. In particular, the surgeon cuts a flap into the skin of the head and holds the flap 51, exposing target biological tissue. The pouch including the medical device is positioned next to the target biological tissue. The pouch can then gently be pressed against the target tissue and illuminated with LED light for several minutes (approximately 3 to 5 minutes) to adhere the pouch to biological tissue.

In this embodiment, the light is white light. The source of the light is an LED has a power output within the range of 10 to 20 W. Reference numeral 2 depicts a beam of light directed to the interface between the cover and the biological tissue.

After sufficient bonding between the pouch and target tissue has occurred, the surgeon can then place the flap of skin 51 over the implant and close the incisions made to seal the implant within the patient's head.

Light can be directed to where the attachment is desired such as where the pouch is contacting biological tissue or tissue-cover interface to selectively crosslink the pouch to the scaffold 2510.

It is envisaged that various sizes and shapes of pouches can be provided to house various sizes and shapes of biomedical implants in other embodiments.

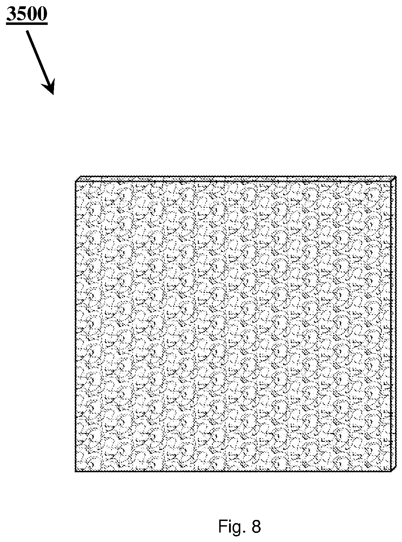

Figure 8 shows an example of the device 3000 in which the cover 3500 is in the form of a sheet. For example, several sheets of different sizes can be provided. Advantageously, the sheet can be cut to a desired size and shape and joined together to provide a custom-made attachment device.

Several sheets can be joined together by a user to surround an implant. For example, an end of a first sheet can be joined to an end of another sheet by bringing the two ends together such that they are in contact and using an electrocautery tool to heat the two ends to a fusing temperature of the biocompatible scaffold to fuse the two sheets together. Alternatively, the two ends of the two sheets can be brought together and water or saline at a fusing temperature can be applied to fuse the two sheets together or solvent fusing can be used as described above.

In another embodiment, the device can include a fastener to fasten parts of a sheet such as one end of a sheet to another end of the same sheet. Alternatively, a fastener can be used to attach part of one sheet to part of another sheet to form a cover.

In use, the sheets or other type of cover is positioned and secured around at least part of the medical device such that the medical device will be retained within the cover after the cover conforms to the outer shape of the medical device.

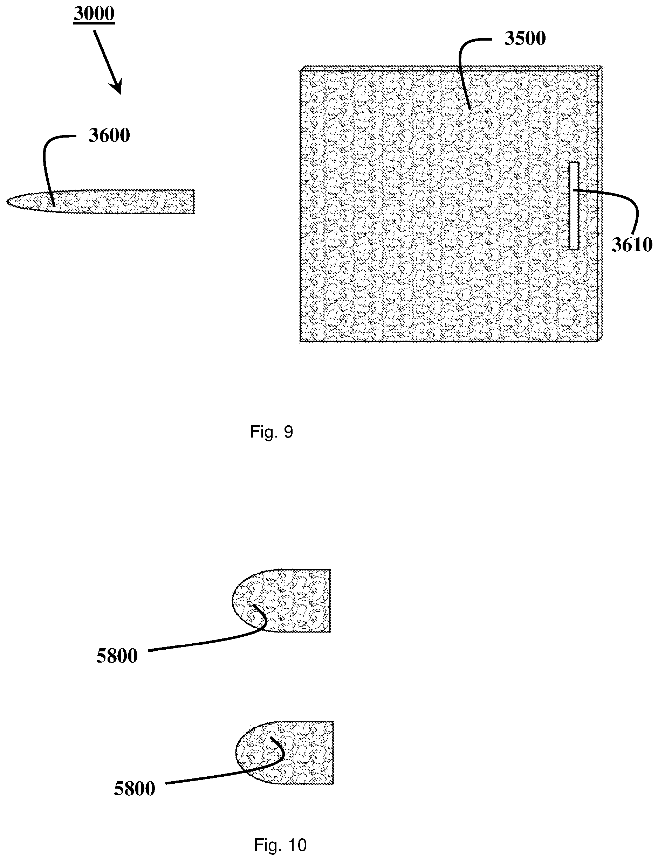

In another embodiment shown in figure 9, a strip of biocompatible scaffold 3600 can be provided as part of the attachment device 3000. The elongated strip 3600 can be fused to one part of the sheet. A slit 3610 can be cut into another part of the sheet for example, near another, opposing end of the sheet and the elongated strip 3600 inserted into the slit 3610 to form an enclosure around a medical device (not shown). Part of the inserted strip can be fused to part of the biocompatible scaffold of the attachment device to prevent the strip from coming out of the slit during use, for example.

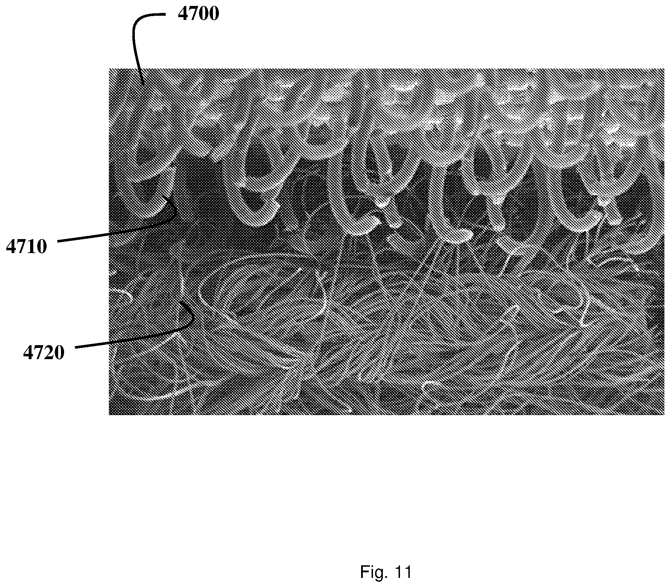

In another embodiment, the fastener can be a biocompatible "hook and loop" type fastener. Figure 11 depicts a biocompatible "hook and loop" type fastener 4700 made of biocompatible material. A first surface layer of a first part 4710 of the fastener can comprise a layer of curled hooks. A second surface layer of the second part 4720 of the fastener comprises loops configured to engage with the hooks of the second surface layer. These hooks can be formed in a specifically shaped silicone template using conventional templating methods known in the art to produce elongate filaments extending perpendicularly to a sheet of material. The tips of these filaments are then heat treated to curl the filaments such that they sufficiently grip the surface of a biocompatible scaffold (not shown). The hook and loop type fastener 4700 can be made of PCL. Similarly, the looped surfaces can be formed using a suitable templating method.

An advantage of having a fastener made of the same type of material as the biocompatible scaffold is that one can fuse the fastener to a cover using an electrocautery tool or by applying heated saline.

As mentioned above, the lowest temperature at which the biocompatible scaffold material of each sheet begins to melt (or melting point) at the location of the biocompatible scaffold that is to be fused is a fusing temperature at which the respective ends of the two sheets can be fused together.

Figure 10 illustrates another embodiment of the device including a biocompatible anchor 5800 which can be used to attach to biological tissue and the biocompatible scaffold. In other words, the biocompatible anchor 5800 is configured to anchor the biocompatible scaffold to the biological tissue of a subject, in use. In this embodiment, the biocompatible anchor is made of the same type of biocompatible scaffold as the biocompatible scaffold of the body of the attachment device. Therefore, the biocompatible anchor is also able to fuse to the biocompatible scaffold of the body at a similar fusing temperature of the biocompatible scaffold.

In this embodiment, the biocompatible anchor 5800 is a relatively small piece of biocompatible scaffold. In other embodiments the anchor can be made in shapes other than the illustrated embodiment such as a circular shape, curved shape, rectangular shape or a combination of these shapes. The anchor 5800 is made of the same type of scaffold as the sheet. The biocompatible anchor 5800 is also coated with a solution comprising photo-active cross-linking agent such as chitosan to from cross links with biological tissue, in use. The biocompatible anchor can have a coating comprising a combination of photoactive dyes as discussed above. In another embodiment, the biocompatible anchor has a coating comprising chitosan and methyl blue. In other embodiments, a solution comprising chitosan and indocyanine green can be used to coat the biocompatible scaffold.

In an embodiment (not shown), the biocompatible anchor 5800 can be a type of biocompatible "hook and loop" fastener as described above in which one part of (either the first or the second part) the "hook and loop" fastener comprises a photoactive crosslinking agent and a photoactive dye so that the anchor can be attached to biological tissue. For example, one part of the anchor can have a surface layer including hooks. This part can be configured to crosslinking with biological tissue. A second part of the anchor comprising biocompatible scaffold can be attached to a cover, for example, by fusing as described above, and be configured to retainably engage with the first party via a surface layer including loops.

Advantageously, one or more of the biocompatible anchors 5800 can be attached to biological tissue at a location where the implant is to be implanted. Thus, the biocompatible anchors can act as markers to provide a guide for the positioning of the implant during surgery, for example. It is envisaged that the biocompatible anchors can be used in surgical planning.

As the anchors are relatively smaller than the medical device to be implanted, they are less bulky and therefore, easier for, for example, a surgeon to manipulate during surgery and for the surgeon to illuminate the tissue-anchor interface to cause crosslinking. The cover can be heat treated such that it conforms to the outer shape of the medical device and then, the cover can be attached to the anchors via fusing as described above.

An advantage of this is that the biocompatible scaffold is then, not required to have much or any photo active crosslinking agent. Crosslinking stiffens the scaffold. Hence, the greater the amount of crosslinking to and within the scaffold, the stiffer the scaffold structure becomes after light is applied to the scaffold. This stiffness may cause the scaffold structure to lose its shape and conform less tightly to the outer surface of the medical device. This may cause the medical device to move within the cover after implantation potentially leading to a predominantly acellular fibrous or chronic inflamed fibrous capsule forming around the implant instead of strong bio-integration of biological tissue within the scaffold.

The cover must therefore, fit tightly around the medical device. As mentioned above, to ensure that the cover fits tightly around the medical device yet is able to sufficiently crosslink with biological tissue to form a strong bond, in another embodiment, the cross-linking agent can be selectively applied to parts of the biocompatible scaffold.

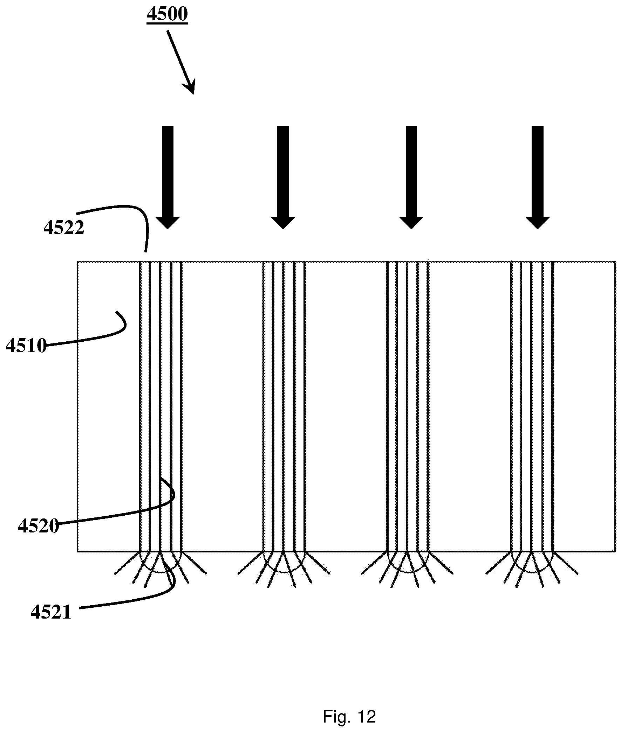

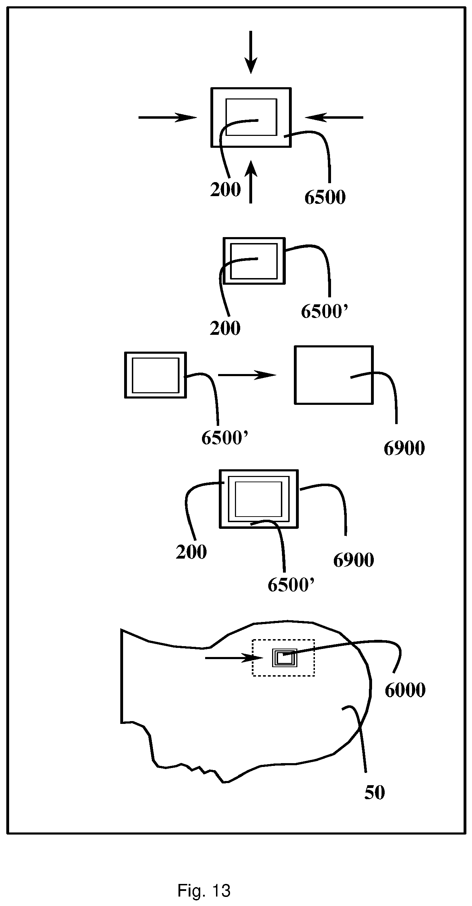

Figure 12 depicts a portion of a cover in which the filaments 4520 are comprised of a light conducting material. Fig 13 is a section taken through a plane extending through the centre of a row of filaments 4520. As shown in Fig 13. Each filament 4520 extends from one side to another side of the scaffold-filament composite.

Each filament is made of a light conducting material. In this embodiment, each filament is made of 45S5 bioactive glass (bioglass). Advantageously, using bioglass within the scaffold in this way allows for enhanced illumination of the tissue-scaffold interface and allows light to better access the tissue-scaffold interface.

Advantageously, bioglass 45S5 has a composition similar to hydroxyapatite which is a mineral component of bone and therefore, provides good osteoconductivity and bioactivity. Bioglass 45S5 is also biodegradeable.

Figure 12 shows how light is conducted through the scaffold to the biological tissue-scaffold interface. Light is directed to the first surface of the scaffold. Tips of the bioglass filaments on the first surface that are illuminated 4522, act as light receiving nodes. As shown via the vertical lines extending through each bioglass filament, the light is conducted substantially linearly through the body of each bioglass filament to the opposite tip 4521 of each filament.

The curvature of the opposite tip 4521 or tip adjacent a tissue-scaffold interface diffracts the light such that a relatively wide area of the tissue-scaffold interface is illuminated to enhance crosslinking of biological tissue to the scaffold. The filaments 4520 serve to focus and guide light through the scaffold without scattering. The diameter, number and spacing between adjacent bioglass filaments 4522 can be varied to sufficiently cover a desired area of a surface of the cover at the tissue-scaffold interface.

Also, in this way, difficult to access parts or surfaces of the cover which cannot be directly illuminated by the surgeon can be indirectly illuminated through the light conducting filaments 4520 to bond the biological tissue to the cover.

In other embodiments, the bioglass filaments can be helical. Advantageously, helical filaments will make the cover less stiff than axial rods. The helical filaments will enhance the ability of the cover to conform to an outer shape of the medical device. In another embodiment, the filament can have a helical shape. The advantage of the helical shape is that the helical filament can be compressed or reduced in length along its central axis which lends flexibility to the cover in the axial direction. However, the helical shape also lends stiffness in the lateral direction.

Figure 13 shows another embodiment of the device 6000. The device 6000 includes a first pouch which is a conformable cover 6500 which does not include photoactive crosslinking material or a photoactive dye. The device 6000 also includes a second pouch 6900. The second pouch 6900 is made of biocompatible scaffold material to allow for tissue integration and includes a photoactive crosslinking agent and photoactive dye. The second pouch 6900 may include strain crystallised filaments but in this embodiment, the second pouch 6900 does not include such filaments. A medical device 200 is placed within the first pouch 6500 which is then sealed. Sufficient heat is then applied to the first pouch 6500 to cause the plurality of biocompatible filaments in the first pouch to contract and cause the cover to substantially conform to the outer shape of the medical device such as by immersing in a saline bath that is at the predetermined temperature. The conformed first pouch is indicated by reference numeral 6500'.

The first conformed pouch 6500' including the medical device 200 is placed into the second pouch 6900. The second pouch 6900 is sealed and then implanted into the patient 50. An incision is made to remove skin tissue at the head of the patient, exposing the target biological tissue. The device 6000 including the conformed first cover 6500' is positioned adjacent the target biological tissue of the subject such that the biocompatible scaffold is in contact with the biological tissue. Light is directed to the interface between the biocompatible scaffold of the second pouch and biological tissue of the second pouch to facilitate crosslinking of the second pouch to the biological tissue.

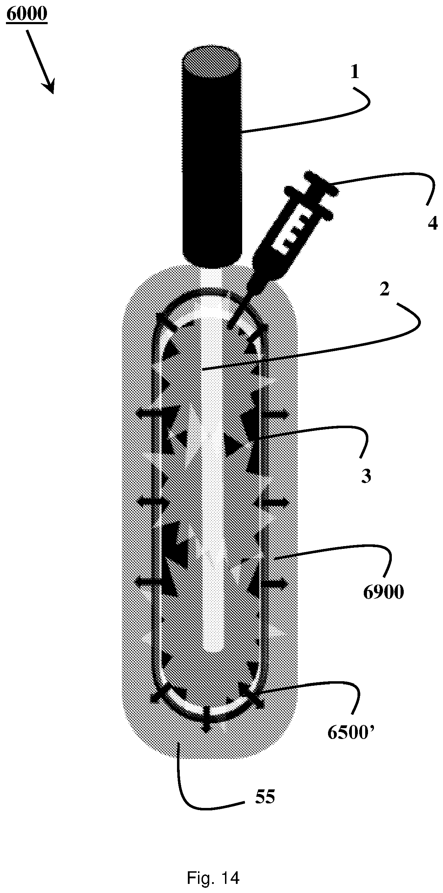

As depicted in figure 14, this method can further include inserting saline 3 into the second pouch using a syringe 4 to cool the biological tissue while a beam of light 2 is directed into the second pouch 6900 from a light source 1. Advantageously, the saline 3 filled second pouch 6900 pushes walls of the second pouch (including photoactive crosslinking material and photoactive dye) 6900 against the target biological tissue 55 and also helps conduct and guide light to the interface between the second pouch 6900 and target biological tissue 55 to enhance crosslinking, especially parts of the interface which cannot be directly illuminated. The arrows indicate the direction of illumination. This allows the surgeon to fix the second pouch 6900 to the target biological tissue 55 without having to excessively maneuver around the device 6000 and the biological tissue or without moving the device 6000 and disrupting the interface.

The attachment devices described herein are configured to secure soft tissue attachment for the first week post-surgery or implantation, until direct tissue attachment and ingrowth through the scaffold occurs. This reduces the likelihood of micromotion of the implant developing early on and causing a fibrous capsule from forming around an implant. The attachment devices described herein will increase the likelihood of abutment of viable soft tissue or hard tissue against the implant resulting in eventual integration within the body of a subject. The attachment devices described herein will minimise the likelihood of a fluid layer and a predominantly acellular fibrous or chronic inflamed fibrous capsule developing around the implant, resulting in rejection of the implant by the body of the subject. Also, advantageously, the crosslinking process liberates oxygen from illuminated parts of the cover which include a photoactive crosslinking dye and agent. Any bacteria present on these illuminated parts will also be killed. Hence, the methods of attaching or connecting a medical device to biological tissue also allow for sterilization of the device.

It is envisaged that the above attachment devices can be used with neurostimulator implants such as cochlear, spinal chord stimulator, deep brain stimulator. The devices can be used in general reconstructive surgery (such as related to soft tissue repair, hernia, oesophageal issues).

It is envisaged that the above attachment devices can be used with orthopaedic implants, autografts, xenografts, meniscal repair (knee surgery).

It is envisaged that the above attachment devices can be used with transcutaneous portal/abutment (Dental implants, BAHA implant, Heart assist devices)

For assisting with autograft or transplant tissue fashioning, adherence, integration in surgery, including mitigating autograft loosening, migration, extrusion.

### Scope of Invention

Thus, while there has been described what are believed to be the preferred embodiments of the invention, those skilled in the art will recognize that other and further modifications may be made thereto without departing from the scope of the appended claims.

### Embodiments:

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

### Comprising and Including

In the claims which follow and in the preceding description of the invention, except where the context requires otherwise due to express language or necessary implication, the word "comprise" or variations such as "comprises" or "comprising" are used in an inclusive sense, i.e. to specify the presence of the stated features but not to preclude the presence or addition of further features in various embodiments of the invention.

Any one of the terms: including or which includes or that includes as used herein is also an open term that also means including at least the elements/features that follow the term, but not excluding others. Thus, including is synonymous with and means comprising.

### Industrial Applicability

It is apparent from the above, that the arrangements described are applicable to the medical device industry.

## Claims

1. A conformable cover (2500) for a medical device comprising:
a porous biocompatible scaffold (2510);
a plurality of biocompatible filaments (2520) embedded within and fixed to the biocompatible scaffold (2510), each of the biocompatible filaments (2520) comprising
a strain crystalized, shape memory polymer or polymer composite and configured to transform from an extended configuration to a reduced configuration when the cover is heated to a predetermined temperature,
wherein, in use, upon covering a medical device (200) with the cover (2500) and heating the cover (2500) to the predetermined temperature, the plurality of biocompatible filaments (2520) is configured within the scaffold to cause the cover (2500) to conform to an outer shape of the medical device (200).

2. The conformable cover (2500) of claim 1, wherein the biocompatible scaffold (2510) comprises a photoactive crosslinking agent for facilitating bonding of the cover (2500) to biological tissue when light is directed on the biocompatible scaffold (2510).

3. The conformable cover (2500) of claim 1, wherein at least one of the plurality of biocompatible filaments (2520) comprises a photoactive crosslinking agent for facilitating bonding of the cover (2500) to biological tissue when light is directed on the cover (2500).

4. An attachment device (2000) for connecting a medical device (200) to biological tissue of a subject, the attachment device (2000) comprising:
a conformable cover (2500) in accordance with claim 1;
the cover (2500) including a photoactive crosslinking agent to facilitate crosslinking of the cover (2500) with biological tissue of a subject when light is directed onto the cover (2500).

5. The attachment device (2000) of claim 4, wherein the biocompatible scaffold (2510) has a hierarchical, porous interconnective structure and is made of polycaprolactone.

6. The attachment device (2000) of claim 4 wherein the predetermined temperature is within the range of 58 to 60 degrees Celsius.

7. The attachment device (2000) of claim 4, wherein the biocompatible scaffold (2510) comprises a density gradient to facilitate integration of soft tissue, a transition to hard tissue and hard tissue within the scaffold (2510).

8. The attachment device (2000) of claim 4 wherein the photoactive crosslinking material is chitosan.

9. The attachment device (2000) of claim 4 wherein the biocompatible scaffold (2510) is coated with photoactive dye.

10. The attachment device (2000) of claim 4 wherein the light is white LED light.

11. The attachment device (2000) of claim 4 wherein the biocompatible filament (2520) comprises a light conductive, bioactive material.

12. The attachment device (2000) of claim 11 wherein each light conductive, bioactive filament (2520) extends through an entire thickness of the cover (2500), between a first surface (2511) and an opposed surface (2512) of the cover (2500) such that one end of each filament (2520) is a light receiving node and the opposed end of the second filament (2520) is located adjacent an interface between biological tissue and the cover (2500), in use.

13. The attachment device of claim 4, further including at least one biocompatible fastener (4700) for fastening one part of the biocompatible scaffold to another part of the biocompatible scaffold or another biocompatible scaffold, to form an enclosure around the medical device, in use.

14. The attachment device of claim 1, further comprising at least one biocompatible anchor (5800) for anchoring the biocompatible scaffold to biological tissue of a subject, in use.

15. The attachment device of claim 14, wherein the biocompatible anchor (5800) comprises biocompatible scaffold, the biocompatible scaffold including a photoactive crosslinking agent on at least one surface of the anchor (5800).

## Patentansprüche

1. Anpassungsfähige Abdeckung (2500) für eine medizinische Vorrichtung, umfassend:
ein poröses biokompatibles Gerüst (2510);
eine Vielzahl von biokompatiblen Filamenten (2520), die innerhalb des biokompatiblen Gerüsts (2510) eingebettet und an diesem fixiert sind, wobei jedes der biokompatiblen Filamente (2520) ein spannungskristallisiertes Formgedächtnispolymer oder - polymerkomposit umfasst und dazu konfiguriert ist, sich aus einer ausgedehnten Konfiguration in eine reduzierte Konfiguration umzuformen, wenn die Abdeckung auf eine vorbestimmte Temperatur erhitzt wird,
wobei die Vielzahl von biokompatiblen Filamenten (2520) dazu konfiguriert ist, im Gebrauch nach Abdecken einer medizinischen Vorrichtung (200) mit der Abdeckung (2500) und Erhitzen der Abdeckung (2500) auf die vorbestimmte Temperatur innerhalb des Gerüsts zu bewirken, dass sich die Abdeckung (2500) an eine äußere Form der medizinischen Vorrichtung (200) anpasst.

2. Anpassungsfähige Abdeckung (2500) nach Anspruch 1, wobei das biokompatible Gerüst (2510) ein photoaktives Vernetzungsmittel zum Erleichtern eines Bondens der Abdeckung (2500) an biologisches Gewebe, wenn Licht auf das biokompatible Gerüst (2510) gerichtet wird, umfasst.

3. Anpassungsfähige Abdeckung (2500) nach Anspruch 1, wobei mindestens eines der Vielzahl von Filamenten (2520) ein photoaktives Vernetzungsmittel zum Erleichtern eines Bondens der Abdeckung (2500) an biologisches Gewebe, wenn Licht auf die Abdeckung (2500) gerichtet wird, umfasst.

4. Anbringungsvorrichtung (2000) zum Verbinden einer medizinischen Vorrichtung (200) mit biologischem Gewebe eines Probanden, wobei die Anbringungsvorrichtung (2000) umfasst:
eine anpassungsfähige Abdeckung (2500) nach Anspruch 1;
wobei die Abdeckung (2500) ein photoaktives Vernetzungsmittel einschließt, um eine Vernetzung der Abdeckung (2500) mit biologischem Gewebe eines Probanden, wenn Licht auf die Abdeckung (2500) gerichtet wird, zu erleichtern.

5. Anbringungsvorrichtung (2000) nach Anspruch 4, wobei das biokompatible Gerüst (2510) eine hierarchische, poröse interkonnektierende Struktur aufweist und aus Polycaprolacton hergestellt ist.

6. Anbringungsvorrichtung (2000) nach Anspruch 4, wobei die vorbestimmte Temperatur innerhalb des Bereichs von 58 bis 60 Grad Celsius liegt.

7. Anbringungsvorrichtung (2000) nach Anspruch 4, wobei das biokompatible Gerüst (2510) einen Dichtegradienten umfasst, um eine Integration von Weichgewebe, einem Übergang zu Hartgewebe und Hartgewebe innerhalb des Gerüsts (2510) zu erleichtern.

8. Anbringungsvorrichtung (2000) nach Anspruch 4, wobei das photoaktive Vernetzungsmaterial Chitosan ist.

9. Anbringungsvorrichtung (2000) nach Anspruch 4, wobei das biokompatible Gerüst (2510) mit photoaktivem Farbstoff beschichtet ist.

10. Anbringungsvorrichtung (2000) nach Anspruch 4, wobei das Licht weißes LED-Licht ist.

11. Anbringungsvorrichtung (2000) nach Anspruch 4, wobei das biokompatible Filament (2520) ein lichtleitendes, bioaktives Material umfasst.

12. Anbringungsvorrichtung (2000) nach Anspruch 11, wobei sich jedes lichtleitende, bioaktive Filament (2520) durch eine gesamte Dicke der Abdeckung (2500) zwischen einer ersten Oberfläche (2511) und einer entgegengesetzten Oberfläche (2512) der Abdeckung (2500) derart erstreckt, dass ein Ende jedes Filaments (2520) ein lichtempfangender Knoten ist und sich das entgegengesetzte Ende des zweiten Filaments (2520) im Gebrauch angrenzend an eine Grenzfläche zwischen biologischem Gewebe und der Abdeckung (2500) befindet.

13. Anbringungsvorrichtung nach Anspruch 4, ferner umfassend mindestens ein biokompatibles Befestigungselement (4700) zum Befestigen eines Teils des biokompatiblen Gerüsts an einem anderen Teil des biokompatiblen Gerüsts oder eines anderen biokompatiblen Gerüsts, um im Gebrauch eine Einfassung um die medizinische Vorrichtung zu bilden.

14. Anbringungsvorrichtung nach Anspruch 1, ferner umfassend mindestens einen biokompatiblen Anker (5800) zum Verankern des biokompatiblen Gerüsts im Gebrauch an biologischem Gewebe eines Probanden.

15. Anbringungsvorrichtung nach Anspruch 14, wobei der biokompatible Anker (5800) ein biokompatibles Gerüst umfasst, wobei das biokompatible Gerüst ein photoaktives Vernetzungsmittel auf mindestens einer Oberfläche des Ankers (5800) einschließt.

## Revendications

1. Un revêtement conformable (2500) pour un dispositif médical comprenant :
un échafaudage biocompatible poreux (2510) ;
une pluralité de filaments biocompatibles (2520) incorporés à l'intérieur et fixés à l'échafaudage biocompatible (2510), chacun des filaments biocompatibles (2520) comprenant un polymère à mémoire de forme cristallisée sous contrainte ou un composite polymère et configuré pour se transformer d'une configuration étendue à une configuration réduite lorsque le couvercle est chauffé à une température prédéterminée, dans lequel, lors de l'utilisation, lors du recouvrement d'un dispositif médical (200) avec le revêtement (2500) et du chauffage du couvercle (2500) à la température prédéterminée, la pluralité de filaments biocompatibles (2520) est configurée à l'intérieur de l'échafaudage pour amener le couvercle (2500) à se conformer à une forme extérieure du dispositif médical (200).

2. Le revêtement conformable (2500) selon la revendication 1, dans lequel l'échafaudage biocompatible (2510) comprend un agent de réticulation photoactif pour faciliter la liaison du revêtement (2500) au tissu biologique lorsque la lumière est dirigée sur l'échafaudage biocompatible (2510).

3. Le revêtement conformable (2500) selon la revendication 1, dans lequel au moins un des multiples filaments biocompatibles (2520) comprend un agent de réticulation photoactif pour faciliter la liaison du revêtement (2500) au tissu biologique lorsque la lumière est dirigée sur le revêtement (2500).

4. Un dispositif de fixation (2000) pour connecter un dispositif médical (200) à un tissu biologique d'un sujet, le dispositif de fixation (2000) comprenant :
un revêtement conformable (2500) selon la revendication 1 ;
le revêtement (2500) comprenant un agent de réticulation photoactif pour faciliter la réticulation du revêtement (2500) avec le tissu biologique d'un sujet lorsque la lumière est dirigée sur le revêtement (2500).

5. Le dispositif de fixation (2000) selon la revendication 4, dans lequel l'échafaudage biocompatible (2510) présente une structure interconnective poreuse hiérarchique et est constitué de polycaprolactone.

6. Le dispositif de fixation (2000) selon la revendication 4, dans lequel la température prédéterminée est comprise entre 58 et 60 degrés Celsius.

7. Le dispositif de fixation (2000) selon la revendication 4, dans lequel l'échafaudage biocompatible (2510) comprend un gradient de densité pour faciliter l'intégration des tissus mous, une transition vers les tissus durs et les tissus durs à l'intérieur de l'échafaudage (2510).

8. Le dispositif de fixation (2000) selon la revendication 4, dans lequel le matériau de réticulation photoactif est le chitosane.

9. Le dispositif de fixation (2000) selon la revendication 4, dans lequel l'échafaudage biocompatible (2510) est revêtu d'un colorant photoactif.

10. Le dispositif de fixation (2000) selon la revendication 4, dans lequel la lumière est une lumière LED blanche.

11. Le dispositif de fixation (2000) selon la revendication 4, dans lequel le filament biocompatible (2520) comprend un matériau bioactif conducteur de lumière.

12. Le dispositif de fixation (2000) selon la revendication 11, dans lequel chaque filament bioactif conducteur de lumière (2520) s'étend sur toute l'épaisseur du revêtement (2500), entre une première surface (2511) et une surface opposée (2512) du revêtement (2500) de telle sorte qu'une extrémité de chaque filament (2520) est un nœud de réception de lumière et l'extrémité opposée du second filament (2520) est située à proximité d'une interface entre le tissu biologique et le revêtement (2500), en cours d'utilisation.

13. Le dispositif de fixation selon la revendication 4, comprenant en outre au moins un élément de fixation biocompatible (4700) pour fixer une partie de l'échafaudage biocompatible à une autre partie de l'échafaudage biocompatible ou à un autre échafaudage biocompatible, pour former une enceinte autour du dispositif médical, en cours d'utilisation.

14. Le dispositif de fixation selon la revendication 1, comprenant en outre au moins une ancre biocompatible (5800) pour ancrer l'échafaudage biocompatible au tissu biologique d'un sujet, en cours d'utilisation.

15. Le dispositif de fixation selon la revendication 14, dans lequel l'ancrage biocompatible (5800) comprend un échafaudage biocompatible, l'échafaudage biocompatible comprenant un agent de réticulation photoactif sur au moins une surface de l'ancrage (5800).
